# EUROPEAN PATENT APPLICATION

(11) **EP 1 961 754 A1**
(43) Date of publication of application: **27.08.2008**
(21) Application number: 06834656.8
(22) Date of filing: 14.12.2006
(51) Int. Cl.: C07D 487/04, A61K 31/5025, A61K 45/00, A61P 1/00, A61P 15/12, A61P 25/00, A61P 25/14, A61P 25/18, A61P 25/24, A61P 43/00

(54) **BICYCLIC HETEROCYCLIC COMPOUND**

(30) Priority: 15.12.2005 JP 2005361617
(71) Applicant: ONO PHARMACEUTICAL CO., LTD., Osaka-shi, Osaka 541-8526 (JP)
(72) Inventor: NAKAI, Hisao, Mishima-gun, Osaka 618-8585 (JP); SAITO, Tetsuji, Mishima-gun, Osaka 618-8585 (JP); KAGAMIISHI, Yoshifumi, Mishima-gun, Osaka 618-8585 (JP)
(74) Representative: Henkel, Feiler & Hänzel
(86) International application number: PCT/JP2006/324902
(87) International publication number: WO 2007/069671

(57) **Abstract**

A compound represented by the formula (I), a salt thereof, an N-oxide thereof, a solvate thereof, or a prodrug thereof: 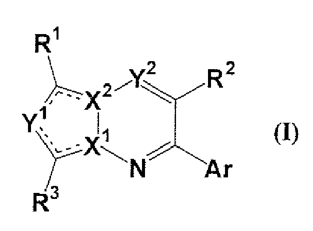 wherein X¹ represents N and X² represents C, or X¹ represents C and X² represents N;
Y¹ represents CR⁴ or N;
Y² represents CH or N;
wherein both Y¹ and Y² do not represent N at the same time;
R¹ represents (1) C3-10 branched alkyl which may be substituted or (2) -(CH₂)ₘ-NR⁵R⁶;R², R³, and R⁴ each independently represent H, C1-4 alkyl, a halo-substituted C1-4 alkyl, C2-4 alkenyl, C2-4 alkynyl, or the like;
R⁵ and R⁶ each independently represent C1-6 alkyl which may be substituted, or R⁵ represents H, and R⁶ represents C3-6 branched alkyl which may be substituted; and
Ar represents an aromatic ring which may be substituted, is useful as a pharmacologically active ingredient having a CRF antagonistic activity in preventing and/or treating neuropsychiatric diseases, diseases of peripheral organs or the like.

## Description

### Technical Field

The present invention relates to a novel bicyclic heterocyclic ring compound or a salt thereof, and a pharmaceutical containing as an active ingredient the same. More specifically, the present invention relates to a novel bicyclic heterocyclic ring compound represented by the formula (I), a salt thereof, an N-oxide thereof, a solvate thereof, or a prodrug thereof, and a pharmaceutical containing as an active ingredient the same: 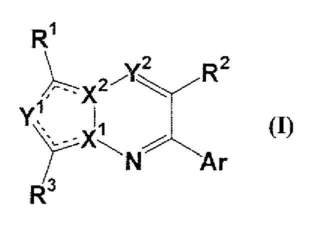 wherein all symbols represent the same meanings as described hereinafter.

### Background Art

Corticotropin Releasing Factor (CRF) is a peptide including 41 amino acid isolated from ovine hypothalamic in 1981. It was suggested that CRF was released from hypothalamic and controlled a secretion of adrenocorticotropic hormone (ACTH) from hypophysis [Science, 218, 377-379 (1982)].

ACTH, which is released by a stimulation of CRF, stimulates a secretion of cortisol from adrenal cortex, and relates to a systemic action for reproduction, growth, gastrointestinal function, inflammation, immune system, nervous system, etc. Consequently, CRF is believed to plays a role as a regulator of these functions. In view of those things, an involvement of CRF with neuropsychiatric diseases, diseases of peripheral organs has received attention.

On the other hand, the depression patients and the anxiety disorder patients increase, and the number also of depression patients with the slight illness increases recently. Moreover, an aged patient is commanding a majority in the depression patient. Under these circumstances, from the earliness of the appearance of the effect and in view of the side effect, neuropsychiatric disease treatment which can be easily used is requested more and more.

Currently, for the treatment of neuropsychiatric diseases, for example, tricyclic antidepressants, tetracyclic antidepressants, monoamine oxidase inhibitors, serotonin and noradrenaline reuptake inhibitors (SNRI), selective serotonin reuptake inhibitors (SSRI), etc. as antidepressant are used. However, the therapeutic gain is not enough; it will take a long time by the time the effect appears; drowsiness, a dryness of the mouth, constipation, difficulty feelings in micturition, etc. are seen as a side effect. As an antianxiety agent, such as benzodiazepine anxiolytic, thienodiazepine anxiolytic, non-benzodiazepine anxiolytic etc. are used. However, the therapeutic gain is not also enough; decrease in mental movement function and decrease in concentration and attention power, drowsiness, stagger, dizziness, headache, amnesia, etc. are seen as a side effect.

As the bicyclic heterocyclic compound, WO 2005/026126 describes that a compound represented by the formula (A) has a CRF antagonistic action: 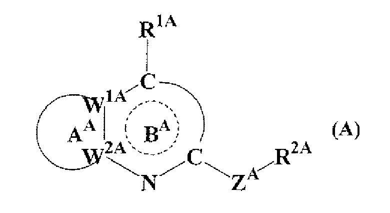 wherein A^{A} ring represents a 5- or 6-membered monocycle which may be substituted with 1 to 3 substituent(s),
B^{A} ring represents a 5-7 membered monocyclic unsaturated heterocycle which may additionally contain or substituted with one or two heteroatoms selected from a nitrogen atom, an oxygen atom, and/or a sulfur atom which may be oxidized other than a nitrogen atom, W^{1A}, and W^{2A},
W^{1A} and W^{2A} each independently represent a carbon atom or a nitrogen atom,
Z^{A} represents -NR^{3A}-, an oxygen atom, a sulfur atom which may be oxidized, or -CR^{4A}R^{5A}-,
R^{1A} represents (i) optionally substituted, C1-15 alkyl, C2-15 alkenyl, or C2-15 alkynyl, (ii) an amino which may be protected, (iii) hydroxyl which may be protected, (iv) mercapto which may be protected, (v) -S(O)_{nA}R^{6A}, (vi) -COR^{7A}, or (vii) a cyclic group which may be substituted, and
R^{2A} represents an unsaturated cyclic group which may be substituted (See Patent Document 1).

In addition, WO 2005/023806 describes that the compound represented by the formula (B) binds to a CRF 1 receptor: 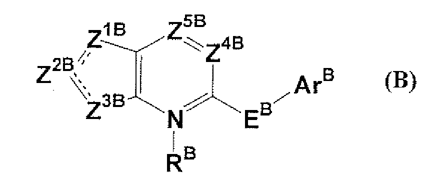 wherein E^{B} is a single bond, and represents O, S(O)_{mB}, NR^{10B}, or CR^{10B}R^{11B};
R^{10B} and R^{11B} independently represent a hydrogen atom or C1-4 alkyl;
m^{B} represents 0,1 or 2; Ar^{B} represents mono-, bi- or tri-substituted phenyl, 1-naphthyl, 2-naphthyl, or heteroaryl;
R^{B} represents an oxygen atom, or nonexistence;
Z^{1B} represents CR^{1B} or CR^{1B}R^{1'B};
represents a nitrogen atom, a oxygen atom, a sulfur atom, CR^{2B}, CR^{2B}R^{2'B}, or NP^{2"B};
Z^{3B} represents a nitrogen atom, an oxygen atom, a sulfur atom, sulfoxide, sulfone, CR^{3B} or CR^{3B}R^{3'B};
represents NR^{B} or CR^{4B};
Z^{5B} represents NR^{B} or CR^{5B};
R^{1B} represents a hydrogen atom, a halogen atom, hydroxy, cyano, amino, alkyl which may be substituted, or the like (see, Patent Document 2).

In WO 2005/028480 describes that the compound represented by the formula (C) is bonded to a CRF receptor 1: 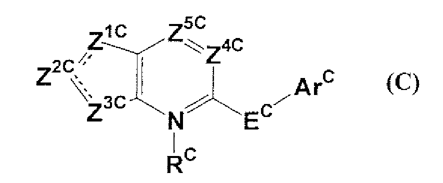 wherein E^{C} represents a single bond, O, S(O)_{mC}, NR^{10C}, or CR^{10C}R^{11C};
Ar^{C} represents mono-, bi- or tri-substituted phenyl, 1-naphthyl, 2-naphthyl, or heteroaryl;
R^{C} represents an oxygen atom or nonexistence;
Z^{1C} represents CR^{1C}, CR^{1C}R^{1'C} or NR^{1"C};
Z^{2C} represents a nitrogen atom or NR^{2"C};
Z^{3C} represents CR^{3C}, CR^{3C}R^{3'C}, a nitrogen atom, NR^{3"C}, an oxygen atom, a sulfur atom, sulfoxide, or sulfone;
Z^{4C} represents NR^{C} or CR^{4C};
Z^{5C} represents NR^{C} or CR^{5C};
R^{1B} represents a halogen atom, hydroxy, cyano, amino, alkyl which may be substituted, or the like (see, Patent Document 3).

[Patent Document 1] WO 2005/026126
[Patent Document 2] WO 2005/023806
[Patent Document 3] WO 2005/028480

### Disclosure of the Invention

### Problem to be solved by the Invention

It is desired to obtain an agent which is easily handled and has potent prevention and/or treatment effects for neuropsychiatric diseases, diseases of peripheral organs or the like.

### Means for solving the Problem

The inventors of the present invention studied intensively in order to solve the above problems, and as a result, found that the object can be achieved by a bicyclic heterocyclic compound.

That is, the present invention relates to:
[1] A compound represented by the formula (I), a salt thereof, an N-oxide thereof, a solvate thereof, or a prodrug thereof: 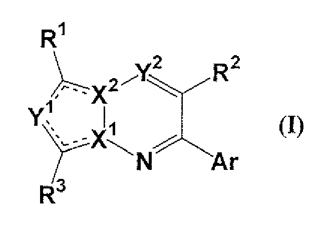 wherein X¹ represents a nitrogen atom and X² represents a carbon atom, or X¹ represents a carbon atom and X² represents a nitrogen atom;
   Y¹ represents CR⁴ or a nitrogen atom;
   Y² represents CH or a nitrogen atom;
   wherein both Y¹ and Y² do not represent nitrogen atoms at the same time: 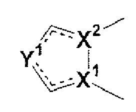 represents 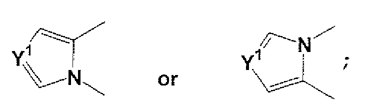
   R¹ represents (1) C3-10 branched alkyl which may be substituted or (2) -(CH₂)ₘ-NR⁵R⁶,
   R² R³, and R⁴ each independently represent a hydrogen atom, C1-4 alkyl which may be substituted with a halogen atom, C2-4 alkenyl, C2-4 alkynyl, nitrile, COOR⁷, CONR⁸R⁹, or a halogen atom,
   R⁵ and R⁶ each independently represent C1-6 alkyl which may be substituted, or R⁵ represents a hydrogen atom, and R⁶ represents C3-6 branched alkyl which may be substituted,
   m represents 0 or an integer of 1-3,
   R⁷ represents a hydrogen atom or C1-4 alkyl,
   R⁸ and R⁹ each independently represent a hydrogen atom or C1-4 alkyl; and
   Ar represents an aromatic ring which may be substituted;
[2] The compound according to [1], a salt thereof, an N-oxide thereof, a solvate thereof, or a prodrug thereof,
   wherein 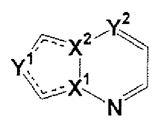 represents the ring represented by one of the following formulae: 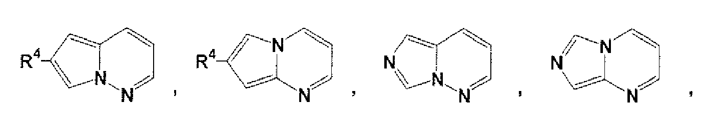 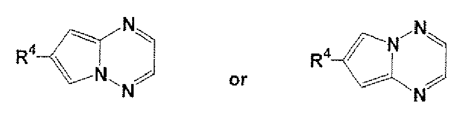 wherein R⁴ represents the same meaning as described in [1];
[3] The compound according to [1], a salt thereof, an N-oxide thereof, a solvate thereof, or a prodrug thereof, wherein R¹ is C3-10 branched alkyl which may be substituted;
[4] The compound according to [1], a salt thereof, an N-oxide thereof, a solvate thereof, or a prodrug thereof, wherein Ar has 1-3 substituent(s), and is a 5-12 membered monocyclic or bicyclic aromatic ring which may contain 1-4 heteroatom(s) selected from a nitrogen atom, an oxygen atom and/or a sulfur atom which may be oxidized;
[5] The compound according to [4], a salt thereof, an N-oxide thereof, a solvate thereof, or a prodrug thereof, wherein Ar is a benzene having 1-3 substituent(s);
[6] The compound according to [1], a salt thereof, an N-oxide thereof, a solvate thereof, or a prodrug thereof, wherein the formula (I) is the formula (I-A): 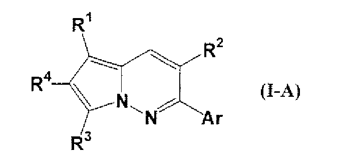 wherein all symbols represent the same meanings as described in [1];
[7] The compound according to [6], a salt thereof, an N-oxide thereof, a solvate thereof, or a prodrug thereof, wherein R¹ represents unsubstituted C3-10 branched alkyl, R² represents C1-4 alkyl, R³ represents C1-4 alkyl, R⁴ represents a hydrogen atom, and Ar represents a benzene having 1-3 substituent(s);
[8] The compound according to [1], a salt thereof, an N-oxide thereof, a solvate thereof, or a prodrug thereof, wherein the compound represented by the formula (I) is:
   (1) 5-(1-ethylpropyl)-2-(4-methoxy-2-methylphenyl)-3,7-dimethylpyrrolo[1,2-b]pyridazine,
   (2) 2-(4-methoxy-2-methylphenyl)-3,7-dimethyl-5-(1-propylbutyl)pyrrolo[1,2-b]pyridazine,
   (3) 5-(1-ethylpropyl)-3,7-dimethyl-2-(2,4,5-trimethylphenyl)pyrrolo[1,2-b]pyridazine,
   (4) 2-(2,4-dimethylphenyl)-5-(1-ethylpropyl)-3,7-dimethylpyrrolo[1,2-b]pyridazine,
   (5) 2-(4-ethoxy-2-ethylphenyl)-5-(2-ethylpropyl)-3,7-dimethylpyrrolo[1,2-b]pyridazine,
   (6) 5-(1-ethylpropyl)-3,7-dimethyl-2-(6-methyl-1,3-benzodioxal-5-yl)pyrrolo[1,2-b]pyridazine,
   (7) 5-[5-(1-ethylpropyl)-3,7-dimethylpyrrolo[1,2-b]pyridazin-2-yl]-N,N,4-trimethyl-2-pyridinamine, or
   (8) 2-(2-ethyl-4,5-dimethoxypbenyl)-5-(1-ethylpropyl)-3,7-dimethylpyrrolo[1,2-b]pyridazine;
[9] A pharmaceutical composition containing as an active ingredient the compound represented by the formula (I) according to [1], a salt thereof, an N-oxide thereof, a solvate thereof, or a prodrug thereof;
[10] The pharmaceutical composition according to [9], which is a CRF antagonist;
[11] The pharmaceutical composition according to [10], which is an agent for preventing and/or treating CRF mediated diseases;
[12] The pharmaceutical composition according to [11], wherein the CRF mediated diseases are neuropsychiatric diseases or digestive diseases;
[13] The pharmaceutical composition according to [12], wherein the neuropsychiatric diseases or the digestive diseases are mood disorder, anxiety disorder, adjustment disorder, stress-related disorder, eating disorder, symptom caused by psychotropic substance or dependency thereon, organic mental disorder, schizophrenic disorder, attention-deficit hyperactivity disorder, irritable bowel syndrome, or gastrointestinal disorder caused by stress;
[14] The pharmaceutical composition according to [13], wherein the mood disorders are depression, bipolar disorder, indefinite complaint, premenstrual dysphoric disorder, postpartum mood disorder, or perimenopausal or menopausal dysphoric disorder, and the anxiety disorders are generalized anxiety disorder, panic disorder, obsessive compulsive disorder, social anxiety disorder, or phobic disorder;
[15] A pharmaceutical composition comprising the compound represented by the formula (I) described in [1], a salt thereof, an N-oxide thereof, a solvate thereof, or a prodrug thereof in combination with at least one kind selected from a tricyclic antidepressant, a tetracyclic antidepressant, a monoamine oxidase inhibitor, a serotonin and noradrenaline reuptake inhibitor, a selective serotonin reuptake inhibitor, a serotonin reuptake inhibitor, a psychostimulant, an antianxiety agent, an antipsychotic agent, a mitochondrial benzodiazepine receptor ligand, a neurokinin 1 antagonist, a gastrointestinal promotility agent, a proton pump inhibitor, a histamine H₂ receptor antagonist, a 5-HT₃ antagonist, a 5-HT₄ agonist, an anticholinergic agent, an antidiarrheal drug, a laxative, and an autonomic modulating agent;
[16] A method of preventing and/or treating CRF mediated diseases, comprising administering to a mammal an effective amount of the compound represented by the formula (I) described in [1], a salt thereof, an N-oxide thereof, a solvate thereof, or a prodrug thereof; and
[17] Use of the compound represented by the formula (I) described in [1], a salt thereof, an N-oxide thereof, a solvate thereof, or a prodrug thereof for manufacturing an agent for preventing and/or treating CRF mediated diseases.

### Effect of the Invention

The bicyclic heterocyclic ring compound of the present invention strongly binds to a CRF receptor to exhibit a strong antagonist action. Therefore, the compound of the present invention is effective for the CRF mediated diseases, for example, neuropsychiatric diseases, or diseases of peripheral organs. In addition, the compound of the present invention has a strong activity in vivo model such as an elevated plus maze test, and is very effective for neuropsychiatric diseases.

### Best Mode for carrying out the Invention

In the present invention, in the ring represent by the following formula, 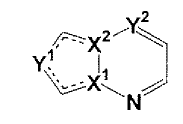 it is preferred that the number of nitrogen atoms in the ring be within three including one which has already been described. In particular, 2 or 3 in total number are preferred. Specifically, the following ring is given:

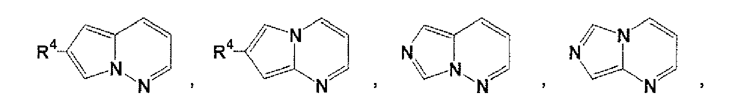

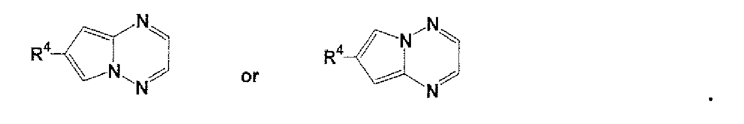

In the present invention, "C3-10 branched alkyl which may be substituted" is "C3-10 branched alkyl substituted with a substituent(s)" or "unsubstituted C3-10 branched alkyl".

In the present invention, "C3-10 branched alkyl" in "C3-10 branched alkyl which may be substituted", "C3-10 branched alkyl substituted with a substituent(s)", and "unsubstituted C3-10 branched alkyl" includes branched propyl, butyl, pentyl, hexyl, octyl, nonyl, and decyl.

Specifically, isopropyl, isobutyl, sec-butyl, tert-butyl, 1-methylbutyl, 1-ethylpropyl, 1,1-dimethylpropyl, 1,2-dimethylpropyl, 2-methylbutyl, 3-methylbutyl, 2,2-dimethylpropyl, 1-methylpentyl, 1-ethylbutyl, 2-ethylbutyl, 1-ethyl-1-methylpropyl, 1-ethyl-2-methylpropyl, 1,1-dimethylbutyl, 1,2-dimethylbutyl, 1,3-dimethylbutyl, 2-methylpentyl, 3-methylpentyl, 4-methylpentyl, 2,3-dimethylbutyl, 1-methylhexyl, 1-ethylpentyl, 2-ethylpentyl, 1-propylbutyl, 2-methyl-3-hexyl, 1,2-dimethylpentyl, 1,3-dimethylpentyl, 1,4-dimethylpentyl, 1-ethyl-1-methylbutyl, 1-methyl-2-ethylbutyl, 1-ethyl-2-methylbutyl, 1-ethyl-3-methylbutyl, 1,1-dimethylpentyl, 1,1,3-trimethylbutyl, 1,1-diethylpropyl, 2-methylhexyl, 3-methylhexyl, 4-methylhexyl, 5-methylhexyl, 3-ethylpentyl, 1-methylheptyl, 2-methylheptyl, 3-methylheptyl, 4-methylheptyl, 5-methylheptyl, 6-methylheptyl, 1-ethylhexyl, 2-ethylhexyl, 3-ethylhexyl, 1-propylpentyl, 2-propylpentyl, 1,5-dimethylhexyl, 1-ethyl-4-methylpentyl, 1-propyl-3-methylbutyl, 1,1-dimethylhexyl, 1-ethyl-1-methylpentyl, 1,1-diethylbutyl, 1-methyloctyl, 2-methyloctyl, 7-methyloctyl, 1-ethylheptyl, 2-ethylheptyl, 1-propylhexyl, 2-propylhexyl, 3-ethylheptyl, 3-propylhexyl, 1-butylpentyl, 1,6-dimethylheptyl, 1-ethyl-5-methylhexyl, 1-propyl-4-methylpentyl, 1-butyl-3-methylbutyl, 1,1-dimethylheptyl, 1-ethyl-1-methylhexyl, 1,1-diethylpentyl, 1-ethyl-1-propylbutyl, 1-methylnonyl, 2-methylnonyl, 1-ethyloctyl, 2-ethyloctyl, 3-ethyloctyl, 1-propylheptyl, 2-propylheptyl, 3-propylheptyl, 1-butylhexyl, 2-butylhexyl, 3-butylhexyl, 1,8-dimethyloctyl, 1-ethyl-6-methylheptyl, 1-propyl-5-methylhexyl, 1-butyl-4-methylpentyl, 1,1-dimethyloctyl, 1-ethyl-1-methylheptyl, 1-ethyl-1-propylpentyl, 1,1-dipropylbutyl, and the like are given.

In the present invention, as the "substituent(s)" in "the C3-10 branched alkyl substituted with a substituent(s)" include hydroxyl, C1-4 alkoxy, a halogen atom, -CF₃, -OCF₃, C3-6 cycloalkyl, -O-(C3-6 cycloalkyl), C5-6 monocyclic unsaturated carbocyclic ring, -O-(C5-6 monocyclic unsaturated carbocyclic ring), a 3-6 membered monocyclic heterocyclic ring, -O-(3-6 membered monocyclic heterocyclic ring). Those substituents may be arbitrary substituted in the C3-10 branched alkyl at substitutable positions, but 1 to 4 substitutable positions are preferred.

In the present invention, "C1-4 alkyl" represents straight or branched C1-4 alkyl, and includes methyl, ethyl, n-propyl, isopropyl, n-butyl, isobutyl, sec-butyl, and sec-butyl.

In the present invention, "C1-6 alkyl which may be substituted represents "C1-6 alkyl substituted with a substituent(s)", or "unsubstituted C1-6 alkyl".

In the present invention, the "C1-6 alkyl" in the "C1-6 alkyl which may be substituted", "C1-6 alkyl substituted with a substituent(s)", and "unsubstituted C1-6 alkyl" represents straight or branched C1-6 alkyl, and includes methyl, ethyl, n-propyl, isopropyl, n-butyl, isobutyl, sec-butyl, sec-butyl, pentyl, hexyl, and isomers thereof.

In the present invention, the "substituent(s)" in the "C1-6 alkyl substituted with a substituent(s) includes hydroxyl, C1-4 alkoxy, a halogen atom, -CF₃, -OCF₃, C3-6 cycloalkyl, -O-(C3-6 cycloalkyl), C5-6 monocyclic unsaturated carbocyclic ring, - O-(C5-6 monocyclic unsaturated carbocyclic ring), a 3-6 membered monocyclic heterocyclic ring, -O-(3-6 membered monocyclic heterocyclic ring). Those substituents may be arbitrary substituted in the C1-8 alkyl at substitutable positions, but 1 to 4 substitutable positions are preferred.

In the present invention, "C3-6 branched alkyl which may be substituted" represents "C3-6 branched alkyl substituted with a substituent(s)" or "unsubstituted C3-6 branched alkyl".

In the present invention, the "C3-6 branched alkyl" in the "C3-6 branched alkyl which may be substituted", "C3-6 branched alkyl substituted with a substituent(s)", and "unsubstituted C3-6 branched alkyl" represents branched propyl, butyl, pentyl, and hexyl. For example, isopropyl, sec-butyl, tert-butyl, 1-methylbutyl, 1-ethylpropyl, 1,1-dimethylpropyl, 1,2-dimethylpropyl, 1-methylpentyl, 1-ethylbutyl, 1-ethyl-1-methylpropyl, 1-ethyl-2-methylpropyl, 1,1-dimethylbutyl, 1,2-dimethylbutyl, and 1,3-dimethylbutyl are given.

In the present invention, as the "substituent(s)" in the "C3-6 branched alkyl substituted with a substituent(s)", hydroxyl, C1-4 alkoxy, a halogen atom, -CF₃, -OCF₃, C3-6 cycloalkyl, -O-(C3-6 cycloalkyl), C5-6 monocyclic unsaturated carbocyclic ring, -0-(C5-6 monocyclic unsaturated carbocyclic ring), a 3-6 membered monocyclic heterocyclic ring, and -0-(3-6 membered monocyclic heterocyclic ring) are given. Those substituents may be arbitrary substituted in the C3-6 branched alkyl at substitutable positions, but 1 to 4 substitutable positions are preferred.

In the present invention, the "halogen atom" includes fluorine, chlorine, bromine and iodine.

In the present invention, the "C1-4 alkyl which may be substituted with a halogen atom" represents "C1-4 alkyl" or "C1-4 alkyl substituted with a halogen".

In the present invention, the "C1-4 alkyl substituted with a halogen atom" is C1-4 alkyl substituted with 1-5 atoms selected from fluorine, chlorine, bromine, and iodine, preferably, C1-4 alkyl substituted with 1-3 same atoms selected from fluorine, chlorine, bromine, and iodine.

In the present invention, "C2-4 alkenyl" includes ethenyl, 1-propenyl, 2-propenyl, 1-butenyl, 2-butenyl, and 3-butenyl.

In the present invention, "C2-4 alkynyl" includes ethynyl, 1-propynyl, 2-propynyl, 1-butynyl, 2-butynyl, and 3-butynyl.

In the present invention, "C1-4 alkoxy" includes methoxy, ethoxy, propoxy, isopropyloxy, butoxy, isobutoxy, sec-butoxy, and tert-butoxy.

In the present invention, "C3-6 cycloalkyl" in the "C3-6 cycloalkyl" and "-O-(C3-6 cycloalkyl) includes cyclopropyl, cyclobutyl, cyclopentyl, and cyclohexyl.

In the present invention, "C5-6 monocyclic unsaturated carbocyclic ring" in the "C5-6 monocyclic unsaturated carbocyclic ring" and "-O-(C5-6 monocyclic unsaturated carbocyclic ring)" means C5-6 unsaturated or a partially saturated monocyclic carbocyclic ring, and for example, cyclopentene, cyclohexene, cyclopentadiene, cyclohexadiene, and benzene are given.

In the present invention, the "3-6 membered monocyclic heterocyclic ring" in the "3-6 membered monocyclic heterocyclic ring" and "-O-(3-6 membered monocyclic heterocyclic ring) means a 3-6 membered saturated, partially saturated or unsaturated monocyclic heterocyclic ring containing 1-2 heteroatom selected from a nitrogen atom, an oxygen atom and/or a sulfur atom which may be oxidized, and for example, oxirane, thiirane, aziridine, oxetane, thietane, azetidine, pyrroline, pyrrolidine, imidazoline, imidazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydroxazole(oxazolidine), dihydroisooxazole, tetrahydroisooxazole(isooxazolidine), dihydrothiazole, tetrahydrothiazole(thiazolidine), dihydroisothiazole, tetrahydroisothiazole(isothiazolidine), dihydrooxazine, tetrahydrooxazine, dihydrothiazine, tetrahydrothiazine, morpholine, thiomorpholine, oxathiane, pyrrole, imidazole, pyrazole, pyridine, pyrazine, pyrimidine, pyridazine, furan, pyran, thiophene, thiopyran, oxazole, isooxazole, thiazole, isothiazole, oxazine, and thiazine are given.

In the present invention, "an aromatic cyclic group which may be substituted" means a 5-12 membered monocyclic or bicyclic aromatic cyclic group being unsubstituted or having 1-3 substituent(s), which may contain 1-4 heteroatom(s) selected from a nitrogen atom, an oxygen atom and/or a sulfur atom which may be oxidized. As the aromatic cyclic group, an aromatic carbocyclic group or an aromatic heterocyclic group containing 1-4 heteroatom(s) selected from a nitrogen atom, an oxygen atom and/or a sulfur atom which may be oxidized, is included.

The "aromatic carbocyclic group" represents a 5-12 membered monocyclic or bicyclic aromatic carbocyclic group, and includes a monocyclic aromatic carbocyclic ring, a bicyclic aromatic carbocyclic ring, or a bicyclic fused ring formed of a monocyclic aromatic carbocyclic ring and an unsaturated or saturated monocyclic carbocyclic ring. For example, benzene, indene, indan, naphthalene, dihydronaphthalene, tetrahydronaphthalene, and azulene rings are given. However, in the case of an indene, indan, dihydronaphthalene, and tetrahydronaphthalene rings, a benzene ring among those rings binds to the following ring:

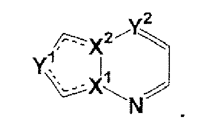

The "aromatic heterocyclic group containing 1-4 heteroatom(s) selected from a nitrogen atom, an oxygen atom and/or a sulfur atom which may be oxidized" represents a 5-12 membered monocyclic or bicyclic aromatic heterocyclic group containing 1-4 heteroatom(s) selected from a nitrogen atom, an oxygen atom and/or a sulfur atom which may be oxidized, and includes a monocyclic aromatic heterocyclic ring, a bicyclic aromatic heterocyclic ring, a bicyclic fused ring formed of a monocyclic aromatic heterocyclic ring and an unsaturated or saturated monocyclic carbocyclic ring, a bicyclic fused ring formed of a monocyclic aromatic carbocyclic ring and an unsaturated or saturated monocyclic heterocyclic ring, or a bicyclic fused ring formed of a monocyclic aromatic heterocyclic ring and an unsaturated or saturated monocyclic heterocyclic ring. For example, pyrrole, imidazole, triazole, pyrazole, pyridine, pyrazine, pyrimidine, pyridazine, furan, thiophene, oxazole, isooxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthylidine, quinoxaline, quinazoline, cinnoline, benzoxazole, benzothiazole, benzimidazole, benzofurazan, benzothiadiazole, benzotriazole, indoline, isoindoline, dihydrobenzofuran, dihydroisobenzofuran, dihydrobenzothiophene, dihydroisobenzothiophene, chromene, chroman, isochroman, tetrahydroquinoline, dihydroquinoline, tetrahydroisoquinoline, dihydroisoquinoline, tetrahydraquinoxaline, dihydroauinoxaline, tetrahydroquinazoline, dihydroquinazoline, and dioxaindan rings are given. However, in the case of the indole, isoindole, benzofuran, isobenzofuran, benzothiophene, isobenzothiophene, indazole, phthalazine, quinoxaline, quinazoline, cinnoline, benzoxazole, benzothiazole, benzimidazole, benzofurazan, benzothiadiazole, benzotriazole, indoline, isoindoline, dihydrobenzofuran, dihydroisobenzofuran, dihydrobenzothiophene, dihydroisobenzothiophene, chromene, chroman, isochroman, and dioxaindan rings, a benzene ring among those rings, or in the case of tetrahydroquinoline, dihydroquinoline, tetrahydroisoquinoline, dihydroisoquinoline, tetrahydroquinoxaline, dihydroquinoxaline, tetrahydroquinazoline, and dihydroquinazoline rings, a pyridine, pyrimidine or pyrazine ring among those rings binds to the following ring:

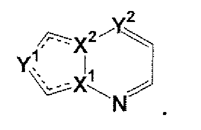

In the present invention, as the "substituents" in the aromatic cyclic group represented by the "aromatic cyclic group which may be substituted", (1) C1-15 alkyl which may be substituted, (2) C2-15 alkenyl which may be substituted, (3) C2-15 alkynyl which may be substituted, (4) hydroxy which may be protected, (5) mercapto which may be protected, (6) amino which may be protected, (7) carbamoyl which may be protected, (8) sulfamoyl which may be protected, (9) carboxyl which may be protected, (10) sulfo (-SO₃H) which may be protected, (11) sulfino (-SO₂H) which may be protected, (12) nitro, (13) cyano, (14) amidino, (15) imino, (16) a halogen atom, (17) a cyclic group which may be substituted, (18) C1-7 acyl, (19) oxo, and (20) thioxo are given. Those substituents may be arbitrary substituted at 1-3 substitutable positions.

In the present invention, C1-15 alkyl which may be substituted represents straight or branched C1-15 alkyl which may be substituted, and for example, methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, or pentadecyl which may be substituted is given.

In the present invention, C2-15 alkenyl which may be substituted represents straight or branched C2-15 alkenyl having 1-3 double bonds and which may be substituted, and for example, vinyl, propenyl, butenyl, pentenyl, hexenyl, hexadienyl, heptenyl, heptadienyl, octenyl, octadienyl, nonenyl, nonadienyl, decenyl, decadienyl, undecenyl, dodecenyl, tridecenyl, tetradecenyl, and pentadecenyl which may be substituted are given.

In the present invention, C2-15 alkynyl which may be substituted represents straight or branched C2-15 alkynyl having 1-3 triple bonds and which may be substituted, and for example, ethynyl, propynyl, butynyl, pentynyl, hexynyl, hexadynyl, heptynyl, heptadynyl, octynyl, octadynyl, nonynyl, decynyl, undecynyl, dodecynyl, tridecynyl, tetradecynyl, and pentadecynyl which may be substituted are given.

In the present invention, "C1-15 alkyl which may be substituted", "C2-15 alkenyl which may be substituted", and "C2-15 alkynyl which may be substituted" each represent "C1-15 alkyl which is substituted by a substituent(s) or unsubstituted", "C2-15 alkenyl which is substituted by a substituent(s) or unsubstituted", and "C2-15 alkynyl which is substituted by a substituent(s) or unsubstituted", and as the "substituent(s)", a group selected from the following substituent group A is given. Those substituents may be substituted at 1-4 substitutable positions.

The substituent group A represents (1) a halogen atom, (2) CF₃, (3) OCF₃, (4) cyano, (5) nitro, (6) hydroxy which may be protected by C1-6 alkyl, C2-6 alkenyl, C2-6 alkynyl, a cyclic group or a protective group having desorption property, (7) C1-7 acyl, (8) carboxyl which may be protected by C1-6 alkyl, C2-6 alkenyl, C2-6 alkynyl, or a cyclic group, (9) carbamoyl which may be protected by C1-6 alkyl, C2-6 alkenyl, C2-6 alkynyl, or a cyclic group, (10) mercapto which may be protected by C1-6 alkyl, C2-6 alkenyl, C2-6 alkynyl, or a cyclic group, (11) NR¹⁰R¹¹, in which R¹⁰ represents (a) a hydrogen atom, (b) C1-6 alkyl, (c) C2-6 alkenyl, (d) C2-6 alkynyl, or (e) a cyclic group; R¹¹ represents (a) a hydrogen atom, (b) C1-6 alkyl, (c) C2-6 alkenyl, (d) C2-6 alkynyl, (e) -COR¹² in which R¹² represents a hydrogen atom, C1-6 alkyl, C2-6 alkenyl, C2-6 alkynyl, or a cyclic group; (f) -COOR¹² in which R¹² represent the same meaning described above; or (g) - CON(R¹⁰)₂ in which two R¹⁰'s each independently represent the same meaning described above, (12) -S(O)ₙR¹³ in which n represents 1 or 2; R¹³ represents a hydrogen atom, C1-6 alkyl, C2-6 alkenyl, or C2-6 alkynyl, or a cyclic group, (13) -COR¹² in which R¹² represents the same meaning described above, and (14) a cyclic group which may be substituted.

Further, C1-6 alkyl, C2-6 alkenyl, and C2-6 alkynyl in the substituent group A may be substituted by a group selected from a substituent group B, the cyclic group described in (14) of the substituent group A may be substituted by a group selected from a substituent group C. Those substituents may be substituted at 1-5 substitutable positions.

The substituent group B represents (1) C1-6 alkoxy, (2) C1-6 alkylthio, (3) a halogen atom, (4) hydroxy which may be protected by C1-6 alkyl, C2-6 alkenyl, C2-6 alkynyl, a cyclic group, a cyclic group-C1-6 alkyl or a protective group having desorption property, (5) CF₃, (6) OCF₃, (7) nitro, (8) cyano, (9) carboxyl, (10) (C1-6 alkoxy)carbonyl, (11) benzyloxycarbonyl, (12) mercapto, (13) amino, (14) C1-6 alkylamino, (15) di(C1-6 alkyl)amino, (16) carbamoyl, (17) N-(C1-6 alkyl)carbamoyl, (18) N,N-di(C1-6 alkyl)carbamoyl, (19) sulfamoyl, (20) N-(C1-6 alkyl)sulfamoyl, (21) N,N-di(C1-6 alkyl)sulfamoyl, (22) C1-7 acyl, and (23) a cyclic group which may be substituted by a group selected from a substituent group D.

As the substituent group C, (1) C1-6 alkyl, (2) C2-6 alkenyl, (3) G2-6 alkynyl, (4) hydroxy which may be protected by C1-6 alkyl, C2-6 alkenyl, C2-6 alkynyl, a cyclic group, or a protective group having desorption property, (5) mercapto which may be protected by C1-6 alkyl, C2-6 alkenyl, C2-6 alkynyl, or the cyclic group, (6) amino which each may be protected by 1-2 groups selected from C1-6 alkyl, C2-6 alkenyl, C2-6 alkynyl, and the cyclic group, (7) carbamoyl which each may be protected by 1-2 groups selected from C1-6 alkyl, C2-6 alkenyl, C2-6 alkynyl, or the cyclic group, (8) sulfamoyl which each may be protected by 1-2 groups selected from C1-6 alkyl, C2-6 alkenyl, C2-6 alkynyl, or the cyclic group, (9) carboxyl which may be protected by C1-6 alkyl, C2-6 alkenyl, C2-6 alkynyl or the cyclic group, (10) nitro, (11) cyano, (12) amidino, (13) a halogen atom, (14) CF₃, (15) OCF₃, (16) C1-7 acyl, (17) oxo, and (18) thioxo are given.

Further, C1-6 alkyl, C2-6 alkenyl, and C2-6 alkynyl in the substituent group C may be substituted with a group selected from the substituent group B, and a cyclic group included in the substituent group C may be substituted by a group selected from the substituent group D.

The substituent group D represents (1) C1-6 alkyl, (2) C2-6 alkenyl, (3) C2-6 alkynyl, (4) C1-6 alkoxy, (5) C1-6 alkylthio, (6) a halogen atom, (7) CF₃, (8) OCF₃, (9) nitro, (10) cyano, (11) hydroxy which may be protected by C1-6 alkyl, C2-6 alkenyl, C2-6 alkynyl, the cyclic group, the cyclic group-C1-6 alkyl, or a protective group having desorption property, (12) carboxyl, (13) (C1-6 alkoxy)carbonyl, (14) benzyloxycarbonyl, (15) mercapto, (16) amino, (17) C1-6 alkylamino, (18) di(C1-6 alkyl)amino, (19) carbamoyl, (20) N-(C1-6 alkyl)carbamoyl, (21) N,N-di(C1-6 alkyl) carbamoyl, (22) sulfamoyl, (23) N-(C1-6 alkyl) sulfamoyl, (24) N,N-di(C1-6 alkyl)sulfamoyl, (25) C1-7 acyl, (26) oxo, and (27) thioxo.

In the present invention, as the hydroxy which may be protected, for example, (a) C1-15 alkyl which may be substituted, (b) C2-15 alkenyl which may be substituted, (c) C2-15 alkynyl which may be substituted, (d) a cyclic group which may be substituted, or (e) hydroxy protected by a protective group having desorption property or hydroxy which is not protected is given. In this case, as the protective group having desorption property, for example, trityl, methoxymethyl(MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxy carbonyl (Alloc), 2,2,2-trichloroethoxycarbonyl (Troc) are given. Further, hydroxy protected by C1-15 alkyl which may be substituted means C1-15 alkoxy which may be substituted.

In the present invention, mercapto which may be protected represents (a) C1-15 alkyl which may be substituted, (b) C2-15 alkenyl which may be substituted, (c) C2-15 alkynyl which may be substituted, or (d) mercapto protected by a cyclic group which may be substituted or unprotected mercapto.

In the present invention, amino which may be protected represents amino protected by the following 1-2 protective groups or unprotected amino. As the protective groups for the amino, (a) C1-15 alkyl which may be substituted, (b) C2-15 alkenyl which may be substituted, (c) C2-15 alkynyl which may be substituted, (d) a cyclic group which may be substituted, (e) -COR¹⁴ in which R¹⁴ represents a hydrogen atom, C1-15 alkyl which may be substituted, C2-15 alkenyl which may be substituted, C2-15 alkynyl which may be substituted, or a cyclic group which may be substituted, (f) -COOR¹⁴ in which R¹⁴ represents the same meaning described above, and (g) -CON(R¹⁵)₂ in which two R¹⁵'s each independently represent, a hydrogen atom, C1-15 alkyl which may be substituted, C2-15 alkenyl which may be substituted, or C2-15 alkynyl which may be substituted are given.

In the present invention, as the "protective group" in the "carbamoyl which may be protected", "sulfamoyl which may be protected", "carboxyl which may be protected", "sulfo which may be protected", and "sulfino which may be protected", (a) C1-15 alkyl which may be substituted, (b) C2-15 alkenyl which may be substituted, (c) C2-15 alkynyl which may be substituted, or (d) a cyclic group which may be substituted are given.

In the present invention, C1-7 acyl represents, for example, formyl, acetyl, propanoyl, pivaloyl, or benzoyl.

In the present invention, the cyclic group represents a carbocyclic group or a heterocyclic group.

The carbocyclic group represents a C3-12 totally saturated, a partially saturated, or a totally unsaturated monocyclic or bicyclic carbocyclic group, and, for example, cyclopropane, cyclobutane, cyclopentane, cyclohexane, cycloheptane, cyclopentene, cyclohexene, cycloheptene, cyalopentadiene, cyclohexadiene, cycloheptadiene, benzene, pentalene, perhydropentalene, azulene, perhydroazulene, indene, perhydroindene, indan, naphthalene, dihydronaphthalene, tetrahydronaphthalene, perhydronaphthalene, heptalene, and perhydroheptalene rings are given.

The heterocyclic group represents a 3-12 membered totally saturated, partially saturated, or totally unsaturated monocyclic or bicyclic heterocyclic group containing 1-4 heteroatom(s) selected from a nitrogen atom, an oxygen atom and/or a sulfur atom which may be oxidized, and, for example, oxirane, thiirane, aziridine, oxetane, thietane, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, dihydrooxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydroxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, and benzotriazole rings are given.

In the present invention, the cyclic group which may be substituted represents a carbocyclic group or a heterocyclic group which each may be substituted with 1-5 groups selected from a substituent group C. As the carbocyclic group and a heterocyclic group, the cyclic groups described above are given.

In the present invention, C1-6 alkyl represents, for example, methyl, ethyl, n-propyl, isopropyl, n-butyl, isobutyl, sec-butyl, tert-butyl, pentyl, hexyl, and isomers thereof.

In the present invention, C2-6 alkenyl represents, for example, vinyl, propenyl, butenyl, pentenyl, hexenyl, hexadienyl, and isomers thereof.

In the present invention, C2-6 alkynyl represents, for example, ethynyl, propynyl, butynyl, pentynyl, hexynyl, hexadynyl and isomers thereof.

In the present invention, hydroxy protected with C1-6 alkyl means C1-6 alkoxy.

In the present invention, C1-6 alkoxy represents, for example, methoxy, ethoxy, propoxy, isopropoxy, butoxy, isobutoxy, sec-butoxy, tert-butoxy, pentyloxy, or hexyloxy.

In the present invention, C1-15 alkoxy represents straight or branched C1-15 alkoxy, and, for example, methoxy, ethoxy, propoxy, isopropoxy, butoxy, isobutoxy, sec-butoxy, tert-butoxy, pentyloxy, hexyloxy, heptyloxy, octyloxy, nonyloxy, decyloxy, undecyloxy, dodecyloxy, tridecyloxy, tetradecyloxy, or pentadecyloxy is given.

In the present invention, C1-6 alkylthio represents, for example, methylthio, ethylthio, propylthio, isopropylthio, n-butylthio, isobutylthio, sec-butylthio, tert-butylthio, pentylthio, or hexylthio.

In the present invention, C1-5 acyl represents, for example, formyl, acetyl, propanoyl, butanoyl, 2-methylpropanoyl, and pivaloyl.

In the present invention, (C1-6 alkoxy)carbonyl represents, for example, methoxycarbonyl, ethoxycarbonyl, propylcarbonyl, isopropylcarbonyl, butoxycarbonyl, isobutoxycarbonyl, sec-butoxycarbonyl, tert-butoxycarbonyl, pentyloxycarbonyl, or hexyloxycarbonyl.

In the present invention, C1-6 alkylamino represents, for example, methylamino, ethylamino, propylamino, isopropylamino, n-butylamino, isobutylamino, sec-butylamino, tert-butylamino, pentylamino, or hexylamino.

In the present invention, di(C1-6 alkyl)amino represents, for example, dimethylamino, diethylamino, dipropylamino, diisopropylamino, dibutylamino, dipentylamino, dihexylamino, or N-methyl-N-ethylamino.

In the present invention, N-(C1-6 alkyl)carbamoyl represents, for example, N-methylcarbamoyl, N-ethylcarbamoyl, N-propylcarbamoyl, N-isopropylcarbamoyl, N-butylcarbamoyl, N-isobutylcarbamoyl, N-(sec-butyl)carbamoyl, N-(tert-butyl)carbamoyl, N-pentylcarbamoyl, or N-hexylcarbamoyl.

In the present invention, N,N-di(C1-6 alkyl)carbamoyl represents, for example, N,N-dimethylcarbamoyl, N,N-diethylcarbamoyl, N,N-dipropylcarbamoyl, N,N-diisopropylcarbamoyl, N,N-dibutylcarbamoyl, N,N-dipentylcarbamoyl, N,N-dihexylcarbamoyl, or N-methyl-N-ethylcarbamoyl.

In the present invention, N-(C1-6 alkyl)sulfamoyl represents, for example, N-methylsulfamoyl, N-ethylsulfamoyl, N-propylsulfamoyl, N-isopropylsulfamoyl, N-butylsulfamoyl, N-isobutylsulfamoyl, N-(sec-butyl)sulfamoyl, N-(tert-butyl)sulfamoyl, N-pentylsulfamoyl, or N-hexylsulfamoyl.

In the present invention, N,N-di(C1-6 alkyl)sulfamoyl represents, for example, N,N-dimethylsulfamoyl, N,N-diethylsulfamoyl, N,N-dipropylsulfamoyl, N,N-diisopropylsulfamoyl, N,N-dibutylsulfamoyl, N,N-dipentylsulfamoyl, N,N-dihexylsulfamoyl, or N-methyl-N-ethylsulfamoyl.

In the present invention, the cyclic group-C1-6 alkyl represents a carbocyclic group(C1-6) alkyl or a heterocyclic group(C1-6)alkyl, each representing (C1-6) alkyl substituted with one carbocyclic group or (C1-6) alkyl substituted with one heterocyclic group. The carbocyclic group, heterocyclic group and C1-6 alkyl each represent the same meanings described above.

In the present invention, as the preferred compound represented by the formula (I), a compound represented by the formula (I-A), formula (I-B), formula (I-C), formula (I-D), formula (I-E), or formula (I-F) is given: 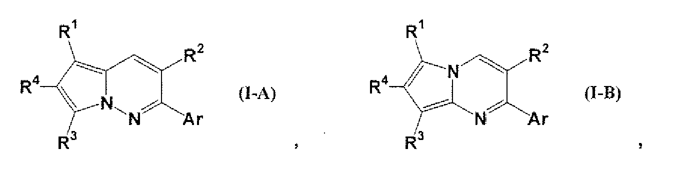 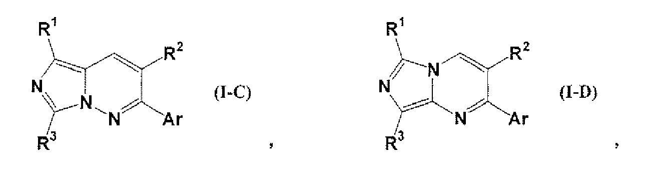 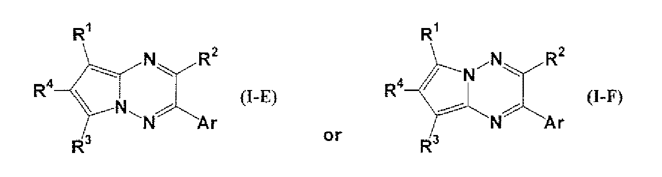 wherein all symbols represent the same meanings described above. As the specific compound, compounds as described in Examples below are given.

In the present invention, it is preferred that R¹ has a branched chain. R¹ preferably includes (1) C3-10 branched alkyl which may be substituted, or (2) -NR⁵R⁶ in which all symbols represent the same meanings described above. In the case of C3-10 branched alkyl which may be substituted, C3-10 branched alkyl whose branch source is a carbon atom at position 1, is more preferred. Particularly preferred is C3-8 branched alkyl which may be substituted, and C3-8 branched alkyl whose branch source is a carbon atom at position 1, is further preferred. Specifically, isopropyl, sec-butyl, tert-butyl, 1-methylbutyl, 1-ethylpropyl, 1,1-dimethylpropyl, 1,2-dimethylpropyl, 1-methylpentyl, 1-ethylbutyl, 1-ethyl-l-methylpropyl, 1-ethyl-2-methylpropyl, 1,1-dimethylbutyl, 1,2-dimethylbutyl, 1,3-dimethylbutyl, 1-methylhexyl, 1-ethylpentyl, 1-propylbutyl, 1,2-dimethylpentyl, 1,3-dimethylpentyl, 1,4-dimethylpentyl, 1-ethyl-1-methylbutyl, 1-methyl-2-ethylbutyl, 1-ethyl-2-methylbutyl, 1-ethyl-3-methylbutyl, 1,1-dimethylpentyl, 1,1,3-trimethylbutyl, 1,1-diethylpropyl, 1-methylheptyl, 1-ethylhexyl, 1-propylpentyl, 1,5-dimethylhexyl, 1-ethyl-4-methylpentyl, 1-propyl-3-methylbutyl, 1,1-dimethylhexyl, 1-ethyl-1-methylpentyl, and 1,1-diethylbutyl are given. Particularly preferably, 1-methylbutyl, 1-ethylpropyl, 1-methylpentyl, 1-ethylbutyl, 1-methylhexyl, 1-ethylpentyl, 1-propylbutyl, 1-methylheptyl, 1-ethylhexyl, and 1-propylpentyl are given. Besides, it is also preferred that in the C3-10 alkyl, the number of carbon atoms of two alkyl groups branched from the carbon at position 1 be the same.

In the present invention, in (1) C3-10 branched alkyl which may be substituted, or (2) -NR⁵R⁶ in which all symbols represent the same meanings described above, being a preferred group as R¹, (1) C3-10 unsubstituted branched alkyl, or C3-10 branched alkyl substituted with 1-2 substituent(s), and (2) - NR R⁵ in which R⁵ and R⁶ each independently represent, C1-6 unsubstituted alkyl, C1-6 alkyl substituted with 1-2 substituent(s), or R⁵ represents a hydrogen atom, and R⁶ represents unsubstituted C3-6 branched alkyl, or C3-6 branched alkyl substituted with 1-2 substituent(s) are more preferred. As the preferred substituents in the "substituted C3-10 branched alkyl", "substituted C1-6 alkyl", and "substituted C3-6 branched alkyl", C1-4 alkoxy, -CF₃, -OCF₃, cyclopropyl, and cyclobutyl are given, and those substituents may be substituted at 1-2 substitutable positions.

In the present invention, preferably R² and R³ each independently represents C1-4 alkyl, C1-4 alkyl substituted with a halogen atom, C2-4 alkenyl, C2-4 alkynyl, nitrile, COOR⁶, or CONR⁷R⁸ in which all symbols represent the same meanings described above. C1-4 alkyl, C2-4 alkenyl, C2-4 alkynyl, nitrile, COOR^{6a} in which R^{6a} represents C1-4 alkyl, or CONR⁷R⁸ in which all symbols represent the same meanings described above, is more referred.

In the present invention, R⁴ preferably represents a hydrogen atom, C1-4 alkyl, or C1-4 alkyl substituted with a halogen atom, and the hydrogen atom is more preferred.

In the present invention, Ar preferably includes, each having 1-3 substituent(s), a 5-12 membered monocyclic aromatic carbocyclic ring, a bicyclic aromatic carbocyclic ring, or a bicyclic fused ring formed of a monocyclic aromatic carbocyclic ring and an unsaturated or saturated monocyclic carbocyclic ring, or, each containing 1-4 heteroatom(s) selected from a nitrogen atom, an oxygen atom and/or a sulfur atom which may be oxidized, a 5-12 membered monocyclic aromatic heterocyclic ring, a bicyclic aromatic heterocyclic ring, or a bicyclic fused ring formed of a monocyclic aromatic heterocyclic ring and an unsaturated or saturated monocyclic carbocyclic ring, a bicyclic fused ring formed of a monocyclic aromatic carbocyclic ring and an unsaturated or a saturated monocyclic heterocyclic ring, or a bicyclic fused ring formed of monocyclic aromatic heterocyclic ring and an unsaturated or saturated monocyclic heterocyclic ring, and more preferably, Ar includes, each having 1-3 substituent(s), benzene, indene, indan, naphthalene, dihydronaphthalene, tetrahydronaphthalene, azulene, pyrrole, imidazole, triazole, pyrazole, pyridine, pyrazine, pyrimidine, pyridazine, furan, thiophene, oxazole, isooxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthylidine, quinoxaline, quinazoline, cinnoline, naphthylidine, benzooxazole, benzothiazole, benzimidazole, benzofurazan, benzothiadiazole, benzotriazole, indoline, isoindoline, dihydrobenzofuran, dihydroisobenzofuran, dihydrobenzothiophene, dihydroisobenzothiophene, chromene, chroman, isochroman, tetrahydroquinoline, dihydroquinoline, tetrahydroisoquinoline, dihydroisoquinoline, tetrahydroquinoxaline, dihydroquinoxaline, tetrahydroquinazoline, dihydroquinazoline, or dioxaindane ring, and further preferably, Ar includes, each having 1-3 substituent(s), benzene, indan, pyridine, pyrimidine, dioxaindan ring. Particularly preferred is a pyridine ring or a benzene ring having 1-3 substituent(s).

As the preferred substituents for Ar described above, (1) C1-15 alkyl which may be substituted, (2) C1-15 alkenyl which may be substituted, (3) hydroxy which may be protected, (4) mercapto which may be protected, (5) amino which may be protected, (6) carbamoyl which may be protected, (7) carboxyl which may be protected, (8) sulfo which may be protected, (9) sulfino which may be protected, (10) cyano, (11) a halogen atom, and (12) a cyclic group which may be substituted are given. Particularly preferred are (1) C1-6 alkyl, (2) C2-6 alkenyl, (3) unsubstituted hydroxy, or hydroxy protected by C1-6 alkyl which may be substituted, or a protective group having desorption property, in which C1-6 alkoxy and trifluoromethoxy are particularly preferred, (4) unsubstituted mercapto, or mercapto protected by C1-6 alkyl which may be substituted, or a protective group having desorption property, in which particularly C1-6 alkylthio is preferred, (5) carboxyl, or carboxyl protected by C1-6 alkyl, or benzyl, (6) cyano, (7) a halogen atom, and (8) a cyclic group which may be substituted. Further, it is preferred that those substituents may be substituted at 1, 2 or 3 substitutable positions of the ring represented by Ar. In particular, in the case where Ar is a 6 membered monocyclic ring, specifically, a benzene or pyridine ring, in the following ring: 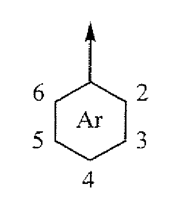 wherein the above ring; 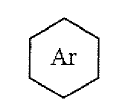 represents a benzene ring or a pyridine ring, and an arrow; 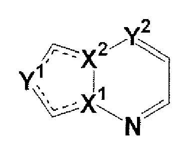 binds to the above ring, the substitution is preferably carried out at (1) position 2, (2) position 3, (3) position 4, (4) positions 2 and 4, (5) positions 2, 4, and 5, or (5) positions 2, 4, and 6.

Unless otherwise specified, all isomers are included in the present invention. For example, alkyl, alkenyl, alkynyl, alkoxy, alkylthio, alkylene, alkenylene and alkynylene include straight and branched isomers. In addition, isomers based on double bond, ring, fused ring (E, Z, cis, trans), isomers resulting from the presence of asymmetric carbon(s) (R, S-configuration, α, β-contiguration, enantiomers, diastereomers), optically active compounds having optical rotation (D, L, d, 1-configuration), polar compounds obtained by chromatographic separations (more polar compound, less polar compound), equilibrium compounds, rotational isomers, the mixtures thereof at any ratio, racemic mixtures are included in the present invention.

In the present invention, as is apparent to the one skilled in the art, unless otherwise indicated, 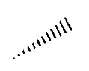 the mark shows that the bond is on the other side of paper (α-configuration), 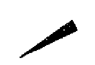 the mark shows that the bond is in front of paper (β-configuration), 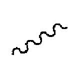 the mark shows that the bond is α-configuration or β-configuration, and 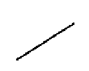 the mark shows that the bond is a mixture of α-configuration and β-configuration.

### [Salts]

The compound represented by the formula (I) may be converted into a salt by known methods. As the salt, pharmaceutically acceptable salts are preferred.

As the salt, alkali metal salt, alkaline earth metal salt, ammonium salt, amine salt, acid addition salt, etc. are given.

Aqueous salt is preferred as the salt. As appropriate salts thereof, salts of alkali metals such as potassium, and sodium; salts of alkaline-earth metals such as calcium and magnesium; ammonium salts such as tetramethylammonium; and salts of pharmaceutically acceptable organic amines such as triethylamine, methylamine, dimethylamine, cyclopentylamine, benzylamine, phenethylamine, piperidine, monoethanolamine, diethanolamine, tris(hydroxymethyl)aminomethane, lysine, arginine, and N-methyl-D-glucamine are given.

Aqueous salts are preferred as the acid addition salts. As appropriate salts thereof, for example, salts of inorganic acids such as hydrochloride, hydrobromide, hydroiodide, sulfate, phosphate, nitrate, or salts of organic acid such as acetate, lactate, tartrate, benzoate, citrate, methanesulfonate, ethanesulfonate, trifluoroacetate, benzenesulfonate, toluenesulfonate, isethionate, glucuronate, or gluconate are given.

Further, N-oxide is included in the salts. The compound of the present invention may be converted into an N-oxide compound by any methods. The N-oxide is the compound in which a nitrogen atom of the compound represented by the formula (I) is oxidized.

The compound represented by the formula (I) and its salt may be replaced with a solvate.

The solvate is preferably nontoxic and aqueous. As appropriate solvates, for example, water or alcohol-based solvents such as ethanol are given.

### [Prodrug]

The prodrug of a compound represented by the formula (I) refers to a compound which is converted into the compound represented by the formula (I) through reaction with an enzyme, a gastric acid, or the like, in the living body. The prodrug of a compound represented by the formula (I) may be exemplified by the compounds represented by the formula (I) where an amino group is contained, in which the amino group has been allowed to undergo acylation, alkylation or phosphorylation (for example, the compounds represented by the formula (I) in which the amino group has been allowed to undergo eicosanoylation, alanylation, pentylaminocarbonylation, (5-methyl-2-oxo-1,3-dioxolen-4-yl)methoxycarbonylation, tetrahydrofuranylation, pyrrolizylmethylation, pivaloyloxymethylation, acetoxymethylation, sec-butylation, etc.); the compounds represented by the formula (I) where a hydroxy group is contained, in which the hydroxy group has been allowed to undergo acylation, alkylation, phosphorylation or boration (for example, the compounds represented by the formula (I) in which the hydroxy group has been allowed to undergo acetylation, palmitoylation, propanoylation, pivaloylation, succinylation, fumarylation, alanylation, dimethylaminomethylcarbonylation, etc.); the compounds represented by the formula (I) where a carboxyl group is contained, in which the carboxyl group has been allowed to undergo esterification or amidation (for example, the compounds represented by the formula (I) in which the carboxyl group has been allowed to undergo ethyl-esterification, isopropyl-esterification, phenyl-esterification, carboxymethyl-esterification, dimethylaminomethyl-esterification, pivaloyloxymethyl-esterification, ethoxycarbonyloxyethyl-esterification, phthalidyl-esterification, (5-methyl-2-oxo-1,3-dioxolen-4-yl)methyl-esterification, cyclohexyloxycarbonylethyl-esterification, methyl-amidation, etc.); the compounds represented by the formula (I) where a carboxyl group is contained, in which the carboxyl group has been allowed to undergo replacement with a hydroxymethyl group, and the like. Those compounds can be produced in accordance with the per se known processes. Besides, the prodrug of the compound represented by the formula (I) may be any of the forms of hydrated or non-hydrated products.

### [Production Process for Compound of the Present Invention]

The compound of the present invention can be produced by the following method, for example.
(1) In the compound represented by the formula (I), the compound represented by the formula (I-A-a): 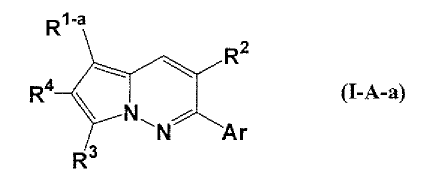
wherein R^{1-a} represents C3-10 alkyl which is branched at a carbon atom on position 1 and may be substituted, and the other symbols represent the same meanings described above, can be produced through the reduction reaction of the compound represented by the formula (II): 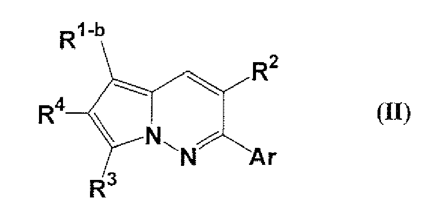 wherein R^{1-b} represents C3-10 branched alkyl which is branched at a carbon atom on position 1 and having one double bond at a carbon atom on position 1, which may be substituted, and the other symbols represent the same meanings described above.

The above reaction is known, and the reaction is carried out, for example, by using a metal catalyst (palladium carbon, etc.) in an organic solvent (ethanol, ethyl, acetate, or tetrahydrofuran, etc.) at room temperature to 80°C under a hydrogen atmosphere.

In the compound represented by the formula (I), a compound represented by the formula (I-B), the formula (I-C), the formula (I-D), the formula (I-E), and the formula (I-F) can be produced by the combination of the known methods.

Compounds represented by the formula (II) can be produced, for example, by a process represented by the following reaction scheme:

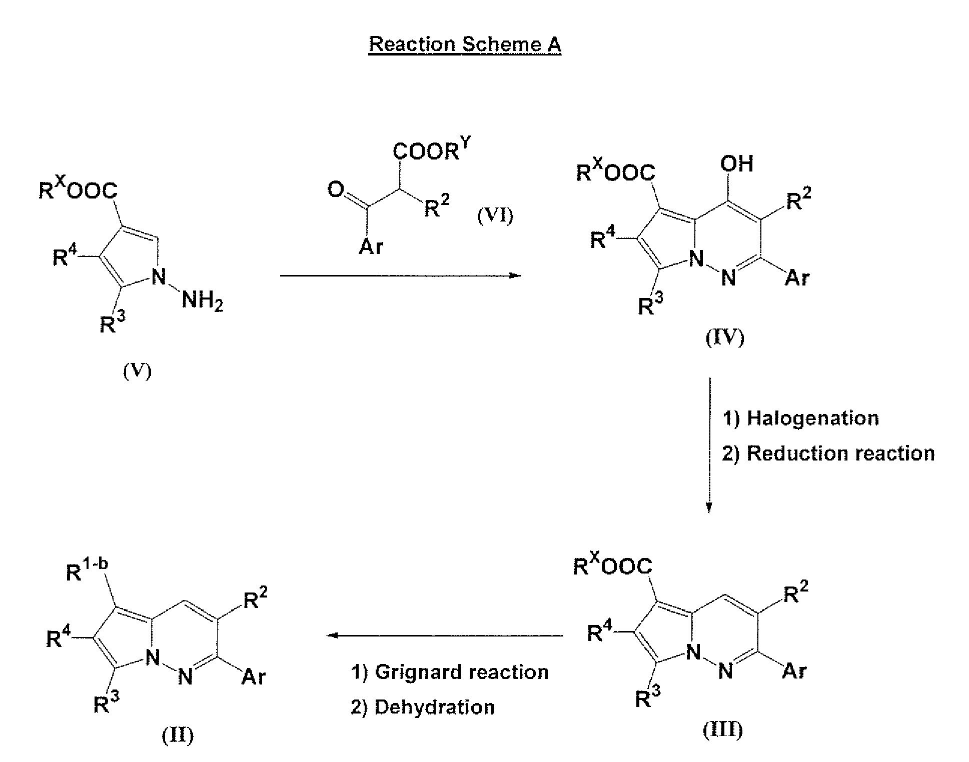

In the reaction scheme A, R^{X} and R^{Y} each independently represent C1-4 alkyl, and the other symbols represent the same meanings described above.

Further, the other starting material and the respective reagents in the present invention are per se known or can be produced in accordance with the known method.

In each reaction in the present specification, reaction products may be purified by general purification techniques, for example, by distillation under atmospheric or reduced pressure, by high performance liquid chromatography, by thin layer chromatography or by column chromatography using silica gel or magnesium silicate; or by washing or by recrystallization. Purification may be carried out after each reaction or after a series of reactions.

### [Toxicity]

The toxicity of the compound of the present invention represented by the formula (I) is very low and therefore, it is confirmed that the compound is sufficiently safe for use as medicine.

### [Application to Pharmaceuticals]

The compound of the present invention represented by the formula (I) binds to a CRF receptor to exhibit antagonist action, and therefore is useful for preventing and/or treating CRF mediated diseases, for example, neuropsychiatric diseases, digestive diseases, respiratory diseases, endocrine diseases, metabolic diseases, cardiovascular diseases, dermatologic diseases, genitourinary diseases, ophthalmologic diseases, or musculoskeletal diseases.

More specifically, as neuropsychiatric diseases, for example, mood disorders (e.g., depression (major depressive disorder (MDD), minor depressive disorder, single episode depression, postpartum depression, child abuse induced depression, elderly depression, masked depression, seasonal depression, recurrent depression), bipolar disorder, indefinite complaint, premenstrual dysphoric disorder, postpartum mood disorder, dysphoric disorder in around the time of climacteric, and perimenopausal dysphoric disorder); anxiety disorders (e.g. generalized anxiety disorder, panic disorder, obsessive compulsive disorder, social anxiety disorders, phobic anxiety disorders (e.g. acrophobia, claustrophobia, agoraphobia, social phobia, etc.)); adjustment disorders (e.g. emotional injury, conduct disorder or disorder with both, physical complaint, isolating oneself from society, occupational stagnant, and stagnant academic achievement); stress-related disorders (e.g. posttraumatic stress disorder (PTSD), stress induced immunosuppression, stress induced headache, stress induced fever, stress induced pain, post operative stress, stress induced gastrointestinal disorder (e.g. gastritis, gastric ulcer, and duodenal ulcer, etc.), irritable bowel syndrome, hyposexuality, and erectile dysfunction)); eating disorders (e.g. anorexia nervosa, binge eating disorder, and nervous vomiting); symptom caused by psychotropic substance or dependency thereon (e.g. alcoholic withdrawal symptoms, alcohol dependence, drug addiction, and drug dependency); organic mental disorder (e.g. senile dementia of Alzheimer's type, and multi-infarct dementia); schizophrenic disorder; attention-deficit hyperactivity disorder; neurodegenerative diseases (e.g. Alzheimer's disease, Parkinson's disease, Huntington's disease, and amyotrophic lateral sclerosis); pain; convulsive disorders (e.g. convulsion and muscle spasm); episodic diseases (e.g. epilepsy, attack and migraine); or sleep disorders (e.g. nonorganic sleep disorder and fiber myalgic sleep disorder) are given. As digestive diseases, for example, peptic ulcer (e.g. gastric ulcer and duodenal ulcer); inflammatory bowel disease (e.g. ulcerative colitis and Crohn's disease); irritable bowel syndrome; stress induced gastrointestinal disorder (e.g. gastritis, gastric ulcer, and duodenal ulcer, etc.); diarrhea; or constipation is given. As respiratory diseases, for example, asthma, bronchitis, chronic obstructive pulmonary disease, or allergic rhinitis is given. As endocrine diseases, for example, disturbed thyroid function, Cushing's disease, or syndrome of inappropriate antidiuretic hormone secretion is given. As metabolic diseases, for example, obesity or hypoglycemia is given. As cardiovascular diseases, for example, hypertension, ischemic heart disease, tachycardia, congestive heart failure, or cerebral vascular disease is given. As dermatologic diseases, for example, atopic dermatitis, allergic contact dermatitis or psoriasis is given. As genitourinary diseases, for example, urinary disturbance, pollakiuria or urinary incontinence is given. As ophthalmologic diseases, for example, uveitis is given. As musculoskeletal diseases, for example, chronic rheumatoid arthritis, osteoarthrosis, or osteoporosis is given.

The compound of the present invention represented by the formula (I) may be administered as a concomitant drug with other medicaments to accomplish the following purposes:
(1) to supplement and/or enhance the preventive and/or therapeutic effect of the present compound;
(2) to improve the kinetics and/or absorption and reduce the dose of the present compound; and/or
(3) to reduce the side effects of the present compound.

A combination of the compound represented by the formula (I) and other medicaments may be administered in the form of the formulations having those components incorporated in one preparation, or may be administered in separate preparations. In the case where those medicaments are administered in separate preparations, they may be administered simultaneously or at different times. Further, in the latter case, the compound of the present invention represented by the formula (I) may be administered before the other medicaments. Alternatively, the other medicaments may be administered before the compound of the present invention represented by the formula (I). The method for the administration of those medicaments are the same or different.

The diseases on which the preventive and/or therapeutic effect of the above combination preparations works are not specifically limited but may be those for which the preventive and/or therapeutic effect of the compound of the present invention represented by the formula (I) is supplemented and/or enhanced.

Examples of other medicaments for supplementing and/or enhancing the preventive and/or therapeutic effect of the compound of the present invention represented by the formula (I) on mood disorders include an antidepressant, a psychoanaleptic, an antianxiety agent, an antipsychotic agent, a mitochondrial benzodiazepine receptor (MBR) ligand, a neurokinin-1 (NK1) antagonist, and the like.

Examples of other medicaments for supplementing and/or enhancing the preventive and/or therapeutic effect of the compound of the present invention represented by the formula (I) on anxiety disorders include an antianxiety agent and a MBR ligand.

Examples of other medicaments for supplementing and/or enhancing the preventive and/or therapeutic effect of the compound of the present invention represented by the formula (I) on irritable bowel syndrome include a gastrointestinal promotility agent, a histamine H₂ receptor antagonist, a proton pump inhibitor, a 5-HT₃ antagonist, a 5-HT₄ agonist, an anticholinergic agent, an antidiarrheal drug, a laxative agent, an autonomic modulating agent, an antidepressant, an antianxiety agent, an MBR ligand, and the like.

As an antidepressant, for example, a tricyclic antidepressant (e.g. amitriptyline hydrochloride, imipramine hydrochloride, clomipramine hydrochloride, dosulepin hydrochloride, nortriptyline hydrochloride, lofepramine hydrochloride, trimipramine maleate, amoxapine); a tetracyclic antidepressant (e.g. maprotiline hydrochloride, mianserin hydrochloride, setiptiline maleate); a monoamine oxidase (MAO) inhibitor (safrazine hydrochloride); serotonin and noradrenaline reuptake inhibitors (SNRI) (e.g. milnacipran hydrochloride, venlafaxine hydrochloride); a selective serotonin reuptake inhibitor (SSRI) (e.g. fluvoxamine maleate, paroxetine hydrochloride, fluoxetine hydrochloride, citalopram hydrochloride); and a serotonin reuptake inhibitor (e.g. trazodone hydrochloride) are given.

As an antianxiety agent, for example, a benzodiazepine anxiolytic (e.g. alprazolam, oxazepam, oxazolam, cloxazolam, clorazepate dipotassium, chlordiazepoxide, diazepam, tofisopam, triazolam, prazepam, fludiazepam, flutazolam, flutoprazepam, bromazepam, mexazolam, medazepam, ethyl loflazepate, lorazepam); a thienodiazepine anxiolytic (e.g. etizolam and clotiazepam); and a non-benzodiazepine anxiolytic (e.g. tandospirone citrate and hydroxylzine hydrochloride) are given.

As a psychoanaleptic, for example, methylphenidate hydrochloride and pemoline are given.

As an antipsychotic agent, for example, sulpiride, trazodone hydrochloride, and serotonin-dopamine antagonist (e.g. risperidone, perospirone hydrochloride hydrate, quetiapine fumarate, and olanzapine) are given.

As a gastrointestinal promotility agent, for example, trimebutine maleate and polycarbophil calcium are given.

As a proton pump inhibitor, for example, omeprazole, lansoprazole, rabeprazole are given.

As a histamine H₂ receptor antagonist, for example, cimetidine, ranitidine, famotidine, nizatidine, lafutidine are given.

As a 5-HT₃ antagonist, for example, alosetron is given.

As a 5-HT₄ agonist, for example, tegaserod, cisapride and mosapride citrate are given.

The mass ratio of the compound represented by the formula (I) and the other medicaments is not specifically limited.

Any combination of two or more kinds of other medicaments may be administered.

Further, the other medicaments for supplementing and/or enhancing the preventive and/or therapeutic effect of the compound represented by the formula (I) include not only those found so far but also those which will be found on the basis of the above-mentioned mechanism.

For the purpose described above, the compounds of the present invention represented by the formula (I), a non-toxic salt thereof, or a combination of the compounds represented by the formula (I) and other medicaments may be normally administered systemically or locally, usually by oral or parenteral administration.

The doses to be administered are determined depending upon, for example, ages, body weights, symptoms, the desired therapeutic effects, the route of administration, and the duration of the treatment. For the human adult, the dose per person is generally from 1 mg to 1,000 mg, by oral administration, from one to several times per day, and from 0.1 mg to 100 mg, by parenteral administration (preferably, nasal drops, eye drops, or ointments), from one to several times per day, or continuous administration for 1 to 24 hours per day from vein.

As described above, of course, the doses to be used depend upon various conditions. Therefore, there are cases in which doses lower than or greater than the ranges specified above may be used.

In the case where the compound of the present invention represented by the formula (I), or the combination agent of the compound represented by the formula (I) and the other medicaments is administrated, those are used as solid preparations for internal use and liquid preparations for internal use for oral administration as well as preparations for injections, external preparations, suppositories, eye drops, inhalations and the like for parenteral administration.

Examples of the solid preparations for internal use for oral administration include tablets, pills, capsules, powders, granules and the like. The capsules include hard capsules and soft capsules. Further, the tablets include sublingual tablet, oral patch and orally disintegrating tablet.

Such solid preparations for internal use is prepared by a formulation method commonly employed by using one or more active substances without modification, or a mixture of one or more active substances with an excipient (lactose, mannitol, glucose, microcrystalline cellulose, starch, etc.), a binder (hydroxypropylcellulose, polyvinylpyrrolidone, magnesium metasilicate aluminate, etc.), a disintegrating agent (calcium cellulose glycolate, etc.), a lubricant (magnesium stearate, etc.), a stabilizer, a solubilizing agent (glutamic acid, aspartic acid, etc.). Further, if necessary, it may be coated with a coating agent (sucrose, gelatin, hydroxypropylcellulose, hydroxypropylmethylcellulose phthalate, etc.). It may be coated with two or more layers. Moreover, capsules made of an absorbable material such as gelatin are involved in the scope thereof.

The liquid preparations for internal use for oral administration include pharmaceutically acceptable aqueous solutions, suspensions, emulsions, syrups, elixirs and the like. Such liquid preparations are prepared by dissolving, suspending or emulsifying one or more active substances in a diluent commonly employed (purified water, ethanol or a mixture liquid thereof, etc.). Such liquid forms may also further contain humectants, suspending agents, emulsifying agents, sweetening agents, flavoring agents, aroma, preservatives, buffers, and the like.

The dose forms of the external preparations for parenteral administration include ointments, gels, creams, fomentations, patches, liniments, atomized agents, inhalations, sprays, aerosols, eye drops, nasal drops, and the like. Such preparations contain one or more active substances and are prepared by a known method or a commonly employed formulation.

Atomized agents, inhalations, and sprays may contain, in addition to a diluent commonly employed, a stabilizer such as sodium bisulfite, and a buffer for imparting isotonicity such as an isotonic agent such as sodium chloride, sodium citrate or citric acid. Methods for producing a spray are described in detail in, for example, US Pat. No. 2,868,691 and US Pat. No. 3,095,355.

The injections for parenteral administration include solutions, suspensions, emulsions and solid injections to be dissolved or suspended before use. The injection is used by dissolving, suspending or emulsifying one or more active substances in a solvent. The solvent includes, for example, distilled water for injection, physiological saline, vegetable oils, alcohols such as propylene glycol, polyethylene glycol and ethanol, and the combinations thereof. The injection may further contain a stabilizer, a dissolution aid (glutamic acid, aspartic acid, Polysorbate 80 (registered trademark), etc.), a suspending agent, an emulsifier, a soothing agent, a buffer, a preservative, and the like. The injection may be produced by sterilizing at the final step or employing an aseptic process. Alternatively, it is also possible that an aseptic solid product such as a freeze-dried product is produced and sterilized or dissolved in aseptic distilled water for injection or another solvent before use.

Other compositions for parenteral administration include suppositories for colorectal administration, pessaries for vaginal administration, and the like, which contain one or more active substances, and are formulated in accordance with common method.

### [Examples]

Hereinafter, the present invention is described in detail by way of examples, but not limited thereto.

Solvents given in parentheses concerning chromatographic separation and TLC each indicate the elution solvent or the developing solvent employed, and the ratio is expressed in ratio by volume.

Solvents given in parentheses concerning NMR each indicate the solvent employed in measurement.

The nomenclature used in the description of the present invention is based on ACD/Name (registered trademark) (version 6.00, manufactured by Advanced Chemistry Development Inc).

### Example 1: Ethyl 5-methyl-1H-pyrrole-3-carboxylate

Ammonium formate (2.7 g) and 10% palladium carbon (200 mg) were added to ethanol (40 mL) solution of ethyl 2-chloro-5-methyl-1H-pyrrole-3-carboxylate (4.0 g; see, Tetrahedron Letters, Vol. 35, No. 33, 5989-5992 (1994)) under argon gas atmosphere at room temperature, and stirred for 18 hours. The reaction mixture was subjected to filtration with Celite (trade name), the filtrate was concentrated under reduced pressure. The residue is added with water, and was extracted two times with ethyl acetate. The extract was washed by a saturated solution of sodium chloride, and concentrated under reduced pressure after drying with anhydrous magnesium sulfate. The obtained residue was purified by silica gel column chromatography (hexane:ethyl acetate=70:30→45:55), to thereby obtain a title compound (3.0 g) having the following physical properties.
TLC:Rf 0.50 (hexane:ethyl acetate=1:1);
¹H-NMR(300 MHz, CDCl₃):δ 8.10 (brs, 1H), 7.28 (m, 1H), 6.31 (m, 1H), 4.26 (q, J=7.2 Hz, 2H), 2.26 (d, J=0.9 Hz, 3H), 1.33 (t, J=6.9 Hz, 3H).

### Example 2: Ethyl 1-amino-5-methyl-1H-pyrrole-3-carboxylate

Sodium hydride (960 mg) was added gradually to anhydrous dimethylformamide (40mL) solution of the compound (3.1 g) produced in Example 1 under argon gas atmosphere at room temperature, and stirred for 30 minutes. Diethyl ether solution (160 mL) of 0.15 M monochloroamine (see, J. Org. Chem, 2004. 69, 1368-1371) was dropped to the resultant mixture taking 1 hour, and is stirred for 30 minutes. The reaction solution was cooled into 0°C, a saturated sodium thiosulfate was added thereto, and was extracted with diethyl ether. The extracted solution was washed by a saturated solution of sodium chloride, and concentrated under reduced pressure after drying with anhydrous magnesium sulfate. The obtained residue was purified by silica gel chromatography (hexane:ethyl acetate=75:25→50:50), to thereby obtain a title compound (206 g) having the following physical properties. TLC: Rf 0.38 (hexane:ethyl acetate acetate=1:1);
¹H-NMR(300 MHz, CDCl₃):δ 7.26 (d, J=1.8 Hz, 1H), 6.22 (m, 1H), 4.59 (s, 2H), 4.23 (q, J=7.2 Hz, 2H), 2.21 (d, J=1.2 Hz, 3H), 1.31 (t, J=7.2 Hz, 3H).

### Example 3: Methyl 3-(4-methoxy-2-methylphenyl)-2-methyl-3-oxopropanoate

Methyl 2-bromopropanoate (0.3 mL) was added to anhydrous tetrahydrofuran (100 mL) of zinc powder (11 g) under argon gas atmosphere, followed by refluxing by heat for 15 minutes. 4-methoxy-2-dimethylbenzonitrile (5.0 g) was added to the resultant reaction solution, and further methyl 2-bromopropanoate (15 mL) was dropped thereinto taking 40 minutes, followed by refluxing by heat for 1 hour. The resultant reaction solution was cooled into room temperature, and was diluted with tetrahydrofuran (50 mL) and 50% potassium carbonate solution (30 mL), followed by stirring for 30 minutes. The supernatant of the reaction solution was separated, and concentrated under reduced pressure. The residual was poured to water, and was extracted with ethyl acetate. The extracted solution was washed by a saturated saline solution, and concentrated under reduced pressure after drying with anhydrous magnesium sulfate. Methanol (110 mL) and IN hydrochloric acid (33 ml) were added to the residue, and stirred at room temperature for 15 hours. The resultant reaction solution was concentrated under reduced pressure, was poured to water, and was extracted with ethyl acetate. The extracted solution was washed by a saturated solution of sodium chloride, and concentrated under reduced pressure after drying with anhydrous magnesium sulfate. The obtained residue was purified by silica gel chromatography (hexane:ethyl acetate=100:0→71:29), to thereby obtain a title compound (5.4 g) having the following physical properties.
TLC: Rf 0.55 (hexane:ethyl acetate=3:1);
¹H-NMR(300 MHz, CDCl₃): *δ* 7.72 (d, J=9.3 Hz, 1H), 6.81-6.73 (m, 2H), 3.85 (s, 3H), 3.68 (d, J=0.6 Hz, 3H), 2.54 (s, 3H), 1.56 (d, J=0.6 Hz, 3H), 1.45 (dd, J=7.2, 0.3 Hz, 3H).

### Example 4: Ethyl 4-hydroxy-2-(4-methoxy-2-methylphenyl)-3,7-dimethylpyrrolo[1,2-b]-5-carboxylate

p-toluenesulfonate monohydrate (113 g) was added to toluene (60 mL) solution of the compound (1.0 g) produced in Example 2 and the compound (1.4 g) produced in Example 3, followed by refluxing by heat for 24 hours. The reaction solution was cooled into room temperature, and a saturated sodium hydrogen carbonate was poured thereto, followed by filtration. The filtrate was extracted with ethyl acetate. The extracted solution was concentrated under reduced pressure after drying with anhydrous magnesium sulfate. The obtained residue was purified by silica gel chromatography (hexane:ethyl acetate=92:8→50:50), to thereby obtain a title compound (1.3 g) having the following physical properties.
TLC: Rf 0.55 (hexane:ethyl acetate=5:1);
¹H-NMR(300 MHz, CDCl₃):δ 13.03 (s, 1H) , 7.15 (d, J=8.7 Hz, 1H), 6.91 (s, 1H), 6.87-6.79 (m, 2H), 4.40 (q, J=7.2 Hz, 2H), 3.85 (s, 3H), 2.46 (d, J=0.6 Hz, 3H), 2.16 (s, 3H), 1.96 (s, 3H), 1.42 (t, J=7.2 Hz, 3H).

### Example 5: Ethyl 4-chloro-2-(4-methoxy-2-methylphenyl)-3,7-dimethylpyrrolo[1,2-b]pyridazine-5-carboxylate

Triphenylphosphine (1.9 g), carbon tetrachloride (2.1 mL) and Celite (trade name; 190 mg) were added to tetrahydrofuran (24 mL) solution of the compound (855 mg) produced in Example 4 under argon gas atmosphere, followed by refluxing by heat for 3 hours. The obtained reaction mixture was cooled into room temperature, hexane and ethyl acetate were added thereto, and was subjected to filtration with Celite (trade name). The filtrate was concentrated under reduced pressure, and was diluted with ethyl acetate. The diluent was washed by a saturated sodium hydrogen carbonate and a saturated saline sequentially, and concentrated under reduced pressure after drying with anhydrous magnesium sulfate. The obtained residue was purified by silica gel column chromatography (hexane:ethyl acetate=100:0→80:20)), to thereby obtain a title compound (900 mg) having the following physical properties.
TLC: Rf 0.38 (hexane:ethyl acetate=5:1);
¹H-NMR(300 MHz, CDCl₃):δ 7.14 (m, 1H), 7.09 (d, J=0.9 Hz, 1H), 6.86-6.79 (m, 2H), 4.36 (q, J=7.5 Hz, 2H), 3.85 (s, 3H), 2.49 (d, J=0.9Hz, 3H), 2.15 (s, 3H), 2.14 (s, 3H), 1.40 (t, J=7.5 Hz, 3H).

### Example 6: Ethyl 2-(4-methoxy-2-methylphenyl)-3,7-dimethylpyrrolo[1,2-b]pyridazine-5-carboxylate

10% palladium carbon (90 mg) and ammonium formate (900 mg) were added to ethanol (45 mL) solution of the compound (900 mg) produced in Example 5 under argon gas atmosphere, followed by stirring for 1 hour at room temperature. The reaction mixture was subjected to filtration with Celite (trade name), and the filtrate was concentrated under reduced pressure. The obtained residue was added with water, and was extracted with ethyl acetate. The extract was washed by a saturated saline and concentrated under reduced pressure after drying with anhydrous magnesium sulfate. The obtained residue was purified by silica gel column chromatography (hexane:ethyl acetate=100:0→83:11), to thereby obtain a title compound (697 mg) having the following physical properties.
TLC: Rf 0.36 (hexane:ethyl acetate=5:1);
¹H-NMR(300 MHz, CDCl₃):δ 8.28 (d, J=1.2 Hz, 1H), 7. 13 (d, J=8.4 Hz, 1H), 7.04 (d, J=0.9 Hz, 1H), 6.88-6.78 (m, 2H), 4.36 (q, J=6.9 Hz, 2H), 3.84 (s, 3H), 2.50 (d, J=0.9 Hz, 3H), 2.14 (s, 3H), 2.09 (d, J=1.2 Hz, 3H), 1.41 (t, J=6.9 Hz, 3H).

### Example 7: 5-[1-ethyl-1-propenyl]-2-(4-methoxy-2-methylphenyl)-3,7-dimethylpyrrolo[1,2-b]pyridazine

Ethylmagnesium bromide (0.95 mL; 3M diethylether solution) was added to anhydrous tetrahydrofuran (3.2 mL) solution of the compound (320 mg) produced in Example 10 under argon gas atmosphere, followed by stirring for 1 hour at 0°C. Further, ethylmagnesium bromide (0.70 mL) was added thereto, and was stirred for 1 hour at room temperature. 1N hydrochloric acid was added to the resultant reaction solution 0°C, and was poured into water, followed by extracting with ethyl acetate. The extract was washed by a saturated sodium hydrogen carbonate and a saturated saline sequentially, and was concentrated under reduced pressure after drying with anhydrous magnesium sulfate. The obtained residue was purified by silica gel column chromatography (hexane:ethyl acetate=100:0→86:14), to thereby obtain a title compound (300 mg) having the following physical properties.
TLC: Rf 0.60 (hexane:ethyl acetate=5:1);
¹H-NMR(300 MHz, CDCl₃):δ 7.65 (d, J=1.2 Hz, 1H), 7.13 (d, J=8.4 Hz, 1H), 6.86-6.76 (m, 2H), 6.55 (d, J=0.9 Hz, 1H), 5.66 (q, J=6.9 Hz, 1H), 3.83 (s, 3H), 2.52 (q, J=7.2 Hz, 2H), 2.49 (s, 3H), 2.18 (s, 3H), 1.98 (d, J=0.9 Hz, 3H), 1.84 (d, J=6.9 Hz, 3H), 1.05 (t, J=7.2 Hz, 3H).

### Example 8: 5-(1-ethylpropyl)-2-(4-methoxy-2-methylphenyl)-3,7-dimethylpyrrolo[1,2-b]pyridazine

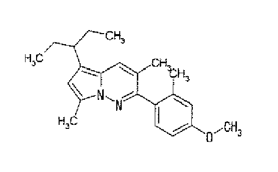

10% palladium carbon (15 mg) was added to ethanol (9 mL) solution of the compound (300 mg) produced in Example 7 at room temperature, followed by stirring for 1 hour under hydrogen gas atmosphere. The reaction mixture was subjected to filtration with Celite (trade name) under argon gas atmosphere, and the filtrate was concentrated under reduced pressure. The obtained residue was purified by silica gel column chromatography (hexane:ethyl acetate=100:0→86:14), to thereby obtain a title compound (284 mg) having the following physical properties.
TLC:Rf 0.60 (hexane:ethyl acetate=5:1);
¹H-NMR(300 MHz, CDCl₃) : δ 0.83 (t, J=7.3 Hz, 6H), 1.50 - 1.83 (m, 4H), 1.96 (s, 3H), 2.19 (s, 3H), 2.50 (s, 3H), 2.55 - 2.68 (m, 1H), 3.83 (s, 3H), 6.41 (s, 1H), 6.75 - 6.84 (m, 2H), 7.14 (d, J=8.1 Hz, 1H), 7.43 (s, 1H).

### Example 8(1): 2-(4-methoxy-2-methylphenyl)-3,7-dimethyl-5-(1-propylbutyl)pyrrolo[1,2-b]pyridazine

The corresponding compound was used, to thereby obtain the following compound having the following physical properties by performing the same process as in Example 8.
TLC: Rf 0.57 (hexane:ethyl acetate=6:1);
¹H-NMR(300 MHz, CDCl₃): δ 0.87 (t, J=7.3 Hz, 6H), 1.14 - 1.33 (m, 4H), 1.49 - 1.74 (m, 4H), 1.97 (s, 3H), 2.19 (s, 3H), 2.50 (s, 3H), 2.73 - 2.90 (m, 1H), 3.84 (s, 3H), 6.42 (s, 1H), 6.75 - 6.88 (m, 2H), 7.16 (d, J=8.4 Hz, 1H), 7.44 (d, J=0.9 Hz, 1H).

### Example 9: 5-(1-ethylpropyl)-2-(4-methoxy-2-methylphenyl)-3-methylpyrrolo[1,2-b]pyridazine

Methyl 1-amino-1H-pyrrolo-3-carboxylate was used in place of the compounds produced in Example 2, to thereby obtain the following compound by performing the same processes as in Example 4→Example 5→Example 6→Example 7→Example 8 in the stated order.
TLC:Rf 0.57 (hexane:ethyl acetate=3:1);
¹H-NMR(300 MHz, CDCl₃) : δ 0.83 (t, J=7.4 Hz, 6H), 1.51 - 1.86 (m, 4H), 1.98 (d, J=1.1 Hz, 3H), 2.18 (s, 3H), 2.55 - 2.72 (m, 1H), 3.83 (s, 3H), 6.61 (d, J=2.7 Hz, 1H), 6.76 - 6.85 (m, 2H), 7.10 - 7.18 (m, 1H), 7.48 (s, 1H), 7.62 (d, J=2.7 Hz, 1H).

### Examples 10(1) to 10(6):

The corresponding ester in place of the compound produced in Exampl 3 was used, to thereby obtain the following compound by performing the same processes as in Example 4→Example 5→Example 6→Example 7→Example 8 in the stated order.

### Example 10(1): 5-(1-ethylpropyl)-3,7-dimethyl-2-(2,4,5-trimethylphenyl)pyrrolo[1,2-b]pyridazine

TLC:Rf 0.48 (hexane:ethyl acetate=20:1);
¹H-NMR(300 MHz, CDCl₃) : δ 0.83 (t, J=7.3 Hz, 6H), 1.54 - 1.67 (m, 2H), 1.68 - 1.82 (m, 2H), 1.97 (s, 3H), 2.13 (s, 3H), 2.26 (s, 3H), 2.28 (s, 3H), 2.50 (s, 3H), 2.56 - 2.67 (m, 1H), 6.42 (s, 1H), 7.01 (s, 1H), 7.06 (s, 1H), 7.45 (s, 1H).

### Example 10(2): 2-(2,4-dimethylphenyl)-5-(1-ethylpropyl)-3,7-dimethylpyrrolo[1,2-b]pyridazine

TLC:Rf 0.64 (hexane:ethyl acetate=9:1);
¹H-NMR(300 MHz, CDCl₃) : δ 0.83 (t, J=7.3 Hz, 6H), 1.50 - 1.84 (m, 4H), 1.97 (d, J=1.1 Hz, 3H), 2.18 (s, 3H), 2.38 (s, 3H), 2.50 (s, 3H), 2.55 - 2.69 (m, 1H), 6.43 (s, 1H), 7.04 - 7.17 (m, 3H), 7.46 (d, J=1.1 Hz, 1H).

### Example 10(3): 2-(4-ethoxy-2-ethylphenyl)-5-(1-ethylpropyl)-3,7-dimethylpyrrolo[1,2-b]pyridazine

TLC:Rf 0.54 (hexane:ethyl acetate=10:1); ¹H-NMR(300 MHz, CDCl₃):δ 0.84 (t, J=7.41 Hz, 6H), 1.15 (t, J=7.50 Hz, 3H), 1.44 (t, J=7.04 Hz, 3H), 1.51 - 1.84 (m, 4H), 1.96 (d, J=0.91 Hz, 3H), 2.44 - 2.55 (m, 5H), 2.57 - 2.69 (m, 1H), 4.08 (q, J=6.95 Hz, 2H), 6.43 (s, 1H), 6.80 (dd, J=8.32, 2.65 Hz, 1H), 6.88 (d, J=2.38 Hz, 1H), 7.33 (d, J=8.23 Hz, 1H), 7.45 (d, J=1.10 Hz, 1H).

### Example 10(4): 5-(1-ethylpropyl)-3,7-dimethyl-2-(6-methyl-1,3-benzodioxol-5-yl)pyrrolo[1,2-b]pyridazine

TLC:Rf 0.51 (hexane:ethyl acetate=10:1);
¹H-NMR(300 MHz, CDCl₃):δ 0.83 (t, J=7.41 Hz, 6H), 1.49 - 1.85 (m, 4H), 1.98 (d, J=0.73 Hz, 3H), 2.11 (s, 3H), 2.51 (s, 3H), 2.56 - 2.70 (m, 1H), 5.97 (s, 2H), 6.44 (s, 1H), 6.73 (s, 1H), 6.76 (s, 1H), 7.46 (d, J=0.92 Hz, 1H).

### Example 10(5): 5-[5-(1-ethylpropyl)-3,7-dimethylpyrralo[1,2-b]pyridazin-2-yl]-N,N,4-trimethyl-2-pyridinamine

TLC:Rf 0.60 (hexane:ethyl acetate=3:1);
¹H-NMR(300 MHz, CDCl₃):δ 0.83 (t, J=7.4 Hz, 6H), 1.57 - 1.67 (m, 2H), 1.68 - 1.79 (m, 2H), 2.03 (d, J=0.9 Hz, 3H), 2.17 (d, J=0.5 Hz, 3H), 2-51 (s, 3H), 2.58 - 2.67 (m, 1H), 3.13 (s, 6H), 6.43 - 6.44 (m, 2H), 7.46 - 7.47 (m, 1H), 8.05 (s, 1H).

### Example 10(6): 2-(2-ethyl-4,5-dimethoxyphenyl)-5-(1-ethylpropyl)-3,7-dimethylpyrrolo[1,2-b]pyridazine

TLC:Rf 0.59 (hexane:ethyl acetate=3:1);
¹H-NMR(300 MHz, CDCl₃) : δ 0.84 (t, J=7.3 Hz, 6H), 1.15 (t, J=7.5 Hz, 3H), 1.51 - 1.85 (m, 4H), 1.98 (s, 3H), 2.39 - 2.54 (m, 2H), 2.52 (s, 3H), 2.57 - 2.70 (m, 1H), 3.85 (s, 3H), 3.94 (s, 3H), 6.44 (s, 1H), 6.74 (s, 1H), 6.84 (s, 1H), 7. 47 (d, J=1.1 Hz, 1H).

### [Pharmacologic Experiment Example]

It was confirmed that the compound of the present invention represented by the formula (I) has a CRF receptor antagonistic activity by the following experiments.

### Experiment Example 1: Binding assay

### [Membrane Preparation]

The forced-expression cell strain of the human CRF receptor I (parent strain is CHO-K1 cell) was cultured until the cell was confluent and collected with a scraper. The collected cells were washed twice with PBS, and were suspended with an ice-colded binding assay buffer (Tris-HCl (50 mM, pH 7.0), EDTA (2 mM, pH 8.0), and MgCl₂ (10 mM)) . After fractured with a dounce-type homogenizer, the suspended cells were centrifuged at 10,000 g, and a membrane fraction was collected. The collected membrane fraction was resuspended with a little amount of the binding assay buffer, thereafter diluted with the binding assay buffer so as to have a concentration of 1 mg/mL. The obtained membrane fraction was used for a binding assay.

### [Binding assay]

¹²⁵I-CRF was diluted with the binding assay buffer so as to have a concentration of 0.5 nM, whereby 50 µL of the diluted ¹²⁵I-CRF was added to a siliconized 1.5-mL tube. Next, test drugs diluted at appropriate times, such as DMSO (for general binding) or 100 µM of CRF (for non-specific binding), was added to a 1 µL tube. Finally, 50 µL of the membrane fraction was added thereto and the reaction was started (final concentration of ¹²⁵I-CRF was 0.25 nM). The tube was incubated at room temperature for 2 hours. After the reaction, the tube was centrifuged at 20,000 g in order to collect the membrane fraction. The supernatant was discarded, and the pellet was washed twice with an ice-colded PBS/0.01% TritonX-100. A membrane binding count was measured using a y counter. The specific binding count was obtained by subtracting the non-specific count from the measured count.

As a result, it was revealed that the compound of the present invention had a strong receptor binding activity (IC₅₀ value < 1 µM). For example, the compound of Example 8 has an IC₅₀ value of 16.4 nM.

### Experiment Example 2: Receptor antagonistic activity (cyclic AMP assay)

The forced expression cell strain of the human CRF receptor I was cultured at 37°C under 5% carbon dioxide and 95% air by using a Ham's F-12 medium (F-12 nutrient mixture) containing 10% bovine fetal serum and 1% antibiotic substance-antifungal agent. On the previous day before the cyclic AMP was measured, the cells were inoculated into a 96-well plate in an amount of 1×10⁴ cell/well. On the measurement day, the cells were washed twice with a Ham's F-12 medium, whereby a solution of Ham's F-12 medium/1 mM 3-isobutyl-1-methylxanthine (assay medium) (178 µL) was added to each well. After the incubation at 37°C for 10 minutes, test drug solutions (2 µL) in various concentrations were added thereto, or DMSO (2 µL) was added to a CRF group and a blank group. An assay medium (20 µL) containing 10 nM human/rat CRF was added to wells of a compound group and the CRF group. An assay medium (20 µL) containing 0.00001% acetic acid was added to the blank group. Each group was further incubated at 37°C for 15 minutes. The supernatant was removed and ice-colded to stop the reaction. Note that the reactions of 2 wells were the same in all reactions. The measurement for accumulated cyclic AMP amount in cells was carried out by using a cyclic AMP Biotrak enzyme immunoassay system (manufactured by Amersham Biosciences Corp.). The accumulated cyclic AMP amounts were calculated by subtracting mean values of corresponding 2 wells in the blank group from that of 2 wells. The IC₅₀ values were calculated with non-linear regression analysis by defining logarithm concentration of the compound as independent variable and an accumulated cyclic AMP amount as dependent variable.

As a result, it was revealed that the compound of the present invention has a strong antagonistic activity against CRF (IC₅₀ value < 1 µM). For example the compound of Example 8 has an IC₅₀ value of 4.9 nM.

### Experiment Example 3: Elevated plus maze test with swim-stressed rat

2 open arms with the same lengths (50 x 10 cm) and 2 close arms (a 30 cm wall was provided) with the same lengths (50 x 10 cm) were arranged in orthogonal manner at a height of 50 cm from the floor to provide an elevated crossroad maze system. In 70 cm above the both open arms, white light was provided, whereby illuminance was maintained at constant.

A 7-week-old SD rat (CHARLES RIVER LABORATORIES JAPAN) was forcedly made to swim for 120 seconds in a pool (40 x 30 x 38 cm) at 22°C of water temperature and 25 cm of water depth. After 9 minutes of soaking stress loading, the rat was put on the center of the system. The activity for 5 minutes of the rat was analyzed with an automatic activity-tracking analysis system (EthoVision Version 3.0, Noldus Information Technology) to calculate the dwell time (second) on the open arm.

Note that no soaking stress was loaded on the control group. In addition, 20% Solutol/HS15 (5 mL/Kg) was orally administered to the vehicle group and test drugs in various concentrations were orally administrated to the test drug administration group 1 hour before the test.

As a result, the dwell time on the open arm was favorably elongated owing to the compound of the present invention at a dose of 10 mg/kg or less compared to that of the vehicle group. For example, administration of 3 mg/g and 10 mg/kg of the compound of Example 8 favorably elongated the dwell time on the open arm compared to that of the vehicle group. It is confirmed that the compound of the present invention has strong effects of preventing and/or treating a psychoneurotic disease, and in particular, an antianxiety effect.

### [Preparation Examples]

### Preparation Example 1:

The following components were mixed by known method, and thereafter formed into a tablet to obtain 10,000 tablets each containing 10 mg of active ingredient per tablet.

| | |
|---|---|
| 5-(1-ethylpropyl)-2-(4-methoxy-2-methylphenyl)-3,7-dimethylpyrrolo[1,2-b]pyridazine | 100 g |
| Carboxymethyl cellulose calcium (disintegrator) | 20 g |
| Magnesium stearate (lubricator) | 10 g |
| Microcrystalline cellulose | 870 g |

### Preparation Example 2:

The following components were mixed by known method, followed by filtration with a dust-removing filter. The resultant filtrate in an amount of 5 mL was loaded into an ampule, and the resultant mixture was sterilized by heat with an autoclave to obtain 10,000 ampules containing 20 mg of active ingredient per ampule.

| | |
|---|---|
| 5-(1-ethylpropyl)-2-(4-methoxy-2-methylphenyl)-3,7-dimethylpyrrolo[1,2-b]pyridazine | 200 g |
| Mannitol | 20 g |
| Distilled water | 50 L |

### Industrial Applicability

The compound of the present invention has a CRF antagonist action, and therefore is effective for preventing and/or treating CRF mediated diseases, for example, neuropsychiatric diseases or digestive diseases.

## Claims

1. A compound represented by the formula (I), a salt thereof, an N-oxide thereof, a solvate thereof, or a prodrug thereof: 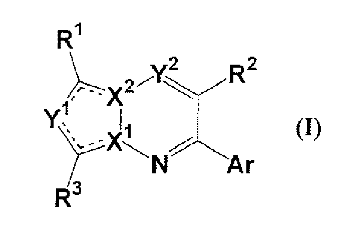 wherein X¹ represents a nitrogen atom and X² represents a carbon atom, or X¹ represents a carbon atom and X² represents a nitrogen atom;
Y¹ represents CR⁴ or a nitrogen atom;
Y² represents CH or a nitrogen atom;
wherein both Y¹ and Y² do not represent nitrogen atoms at the same time: 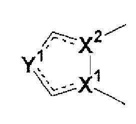 represents 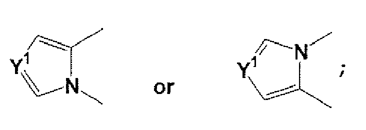
R¹ represents (1) C3-10 branched alkyl which may be substituted or (2) -(CH₂)ₘ-NR⁵R⁶,
R², R³, and R⁴ each independently represent a hydrogen atom, C1-4 alkyl which may be substituted with a halogen atom, C2-4 alkenyl, C2-4 alkynyl, nitrile, COOR⁷, CONR⁸R⁹, or a halogen atom,
R⁵ and R⁶ each independently represent C1-6 alkyl which may be substituted, or R⁵ represents a hydrogen atom, and R⁶ represents C3-6 branched alkyl which may be substituted,
m represents 0 or an integer of 1-3,
R⁷ represents a hydrogen atom or C1-4 alkyl,
R⁸ and R⁹ each independently represent a hydrogen atom or C1-4 alkyl; and
Ar represents an aromatic ring which may be substituted.

2. The compound according to claim 1, a salt thereof, an N-oxide thereof, a solvate thereof, or a prodrug thereof,
wherein 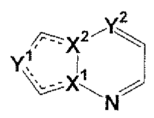 represents the ring represented by one of the following formulae: 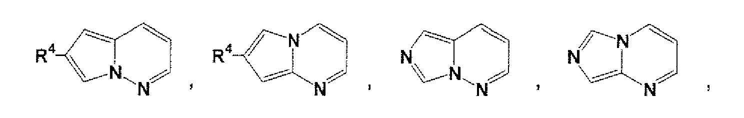 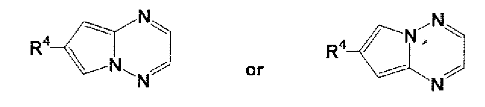 wherein R⁴ represents the same meaning as described in claim 1.

3. The compound according to claim 1, a salt thereof, an N-oxide thereof, a solvate thereof, or a prodrug thereof, wherein R¹ is C3-10 branched alkyl which may be substituted.

4. The compound according to claim 1, a salt thereof, an N-oxide thereof, a solvate thereof, or a prodrug thereof, wherein Ar has 1-3 substituent(s), and is a 5-12 membered monocyclic or bicyclic aromatic ring which may contain 1-4 heteroatom(s) selected from a nitrogen atom, an oxygen atom and/or a sulfur atom which may be oxidized.

5. The compound according to claim 4, a salt thereof, an N-oxide thereof, a solvate thereof, or a prodrug thereof, wherein Ar is a benzene having 1-3 substituent(s).

6. The compound according to claim 1, a salt thereof, an N-oxide thereof, a solvate thereof, or a prodrug thereof, wherein the formula (I) is the formula (I-A): 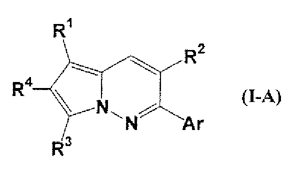 wherein all symbols represent the same meanings as described in claim 1.

7. The compound according to claim 6, a salt thereof, an N-oxide thereof, a solvate thereof, or a prodrug thereof, wherein R¹ represents unsubstituted C3-10 branched alkyl, R² represents C1-4 alkyl, R³ represents C1-4 alkyl, R⁴ represents a hydrogen atom, and Ar represents a benzene having 1-3 substituent(s).

8. The compound according to claim 1, a salt thereof, an N-oxide thereof, a solvate thereof, or a prodrug thereof, wherein the compound represented by the formula (I) is:
(1) 5-(1-ethylpropyl)-2-(4-methoxy-2-methylphenyl)-3,7-dimethylpyrrolo[1,2-b]pyridazine,
(2) 2-(4-methoxy-2-methylphenyl)-3,7-dimethyl-5-(1-propylbutyl)pyrrolo[1,2-b]pyridazine,
(3) 5-(1-ethylpropyl)-3,7-dimethyl-2-(2,4,5-trimethylphenyl)pyrrolo[1,2-b]pyridazine,
(4) 2-(2,4-dimethylphenyl)-5-(1-ethylpropyl)-3,7-dimethylpyrrolo[1,2-b]pyridazine,
(5) 2-(4-ethoxy-2-ethylphenyl)-5-(1-ethylpropyl)-3,7-dimethylpyrrolo[1,2-b]pyridazine,
(6) 5-(1-ethylpropyl)-3,7-dimethyl-2-(6-methyl-1,3-benzodioxol-5-yl)pyrrolo[1,2-b]pyridazine,
(7) 5-[5-(1-ethylpropyl)-3,7-dimethylpyrrolo[1,2-b]pyridazin-2-yl]-N,N,4-trimethyl-2-pyridinamine, or
(8) 2-(2-ethyl-4,5-dimethoxyphenyl)-5-(1-ethylpropyl)-3,7-dimethylpyrrolo[1,2-b]pyridazine.

9. A pharmaceutical composition containing as an active ingredient the compound represented by the formula (I) described in claim 1, a salt thereof, an N-oxide thereof, a solvate thereof, or a prodrug thereof.

10. The pharmaceutical composition according to claim 9, which is a CRF antagonist.

11. The pharmaceutical composition according to claim 10, which is an agent for preventing and/or treating CRF mediated diseases.

12. The pharmaceutical composition according to claim 11, wherein the CRF mediated diseases are neuropsychiatric diseases or digestive diseases.

13. The pharmaceutical composition according to claim 12, wherein the neuropsychiatric diseases or the digestive diseases are mood disorder, anxiety disorder, adjustment disorder, stress-related disorder, eating disorder, symptom caused by psychotropic substance or dependency thereon, organic mental disorder, schizophrenic disorder, attention-deficit hyperactivity disorder, irritable bowel syndrome, or gastrointestinal disorder caused by stress.

14. The pharmaceutical composition according to claim 13, wherein the mood disorders are depression, bipolar disorder, indefinite complaint, premenstrual dysphoric disorder, postpartum mood disorder, or perimenopausal or menopausal dysphoric disorder, and the anxiety disorders are generalized anxiety disorder, panic disorder, obsessive compulsive disorder, social anxiety disorder, or phobic disorder.

15. A pharmaceutical composition comprising the compound represented by the formula (I) described in claim 1, a salt thereof, an N-oxide thereof, a solvate thereof, or a prodrug thereof in combination with at least one kind selected from a tricyclic antidepressant, a tetracyclic antidepressant, a monoamine oxidase inhibitor, a serotonin and noradrenaline reuptake inhibitor, a selective serotonin reuptake inhibitor, a serotonin reuptake inhibitor, a psychostimulant, an antianxiety agent, an antipsychotic agent, a mitochondrial benzodiazepine receptor ligand, a neurokinin 1 antagonist, a gastrointestinal promotility agent, a proton pump inhibitor, a histamine H₂ receptor antagonist, a 5-HT₃ antagonist, a 5-HT₄ agonist, an anticholinergic agent, an antidiarrheal drug, a laxative, and an autonomic modulating agent.

16. A method of preventing and/or treating CRF mediated diseases, comprising administering to a mammal an effective amount of the compound represented by the formula (I) described in claim 1, a salt thereof, an N-oxide thereof, a solvate thereof, or a prodrug thereof.

17. Use of the compound represented by the formula (I) described in claim 1, a salt thereof, an N-oxide thereof, a solvate thereof, or a prodrug thereof for manufacturing an agent for preventing and/or treating CRF mediated diseases.
